# EUROPÄISCHE PATENTANMELDUNG

(11) **EP 3 029 051 A1**
(43) Veröffentlichungstag der Anmeldung: **08.06.2016**
(21) Anmeldenummer: 14196191.2
(22) Anmeldetag: 04.12.2014
(51) Int. Cl.: C07F 9/6574, C07B 41/06, C07C 45/50

(54) **Bisphosphite die eine Naphthyl-Phenyl-Einheit als Flügel-Baustein und eine 2,3'-Bisphenol-Einheit als Zentral-Baustein aufweisen**

(71) Anmelder: Evonik Degussa GmbH, 45128 Essen (DE)
(72) Erfinder: Dyballa, Katrin Marie, 45657 Recklinghausen (DE); Franke, Robert, 45772 Marl (DE); Börner, Armin, 18059 Rostock (DE); Selent, Detlef, 18059 Rostock (DE)

(57) **Zusammenfassung**

Bisphosphite, die eine Naphthyl-Phenyl-Einheit als Flügel-Baustein und eine 2,3'-Biphenol-Einheit als Zentral-Baustein aufweisen. Drei Markush Formeln werden beansprucht, von denen die Formel (I) exemplarisch abgebildet ist.

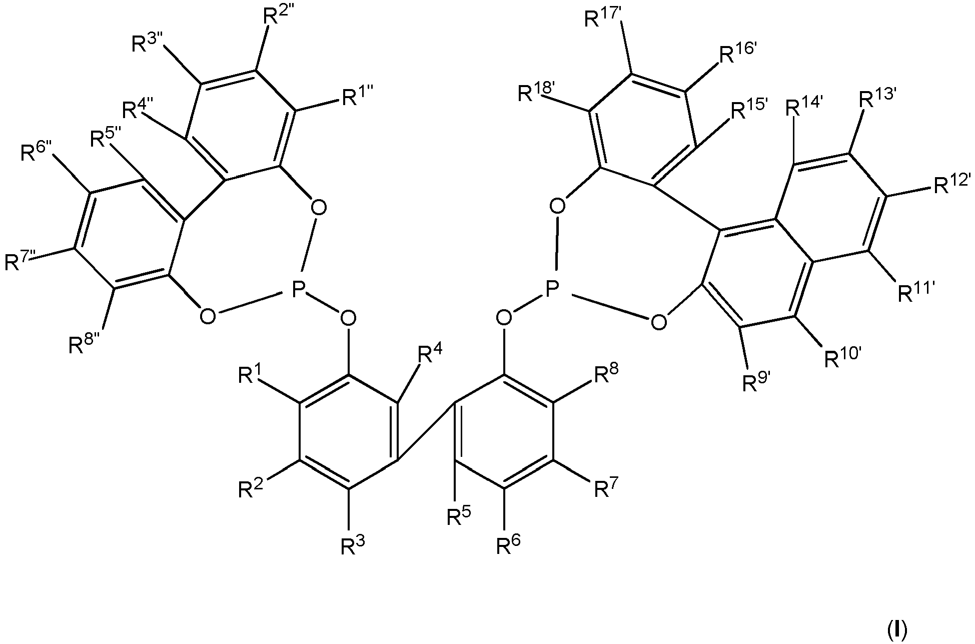

## Beschreibung

Die Erfindung betrifft Bisphosphite, die eine Naphthyl-Phenyl-Einheit als Flügel-Baustein und eine 2,3'-Biphenol-Einheit als Zentral-Baustein aufweisen.

Ein Bisphosphit weist einen Zentral-Baustein, das so genannte Rückgrat, und zwei Flügel-Bausteine auf, welche mit dem Zentral-Baustein über das P-Atom verbunden sind.

Die erfindungsgemäßen Bisphosphite weisen mindestens eine Naphthyl-Phenyl-Einheit als Flügel-Baustein auf. Es ist auch möglich, dass beide Flügel-Bausteine eine Naphthyl-Phenyl-Einheit aufweisen. Der Zentral-Baustein weist eine 2,3'-Biphenol-Einheit auf.

Unter 2,3'-Biphenol-Einheit ist eine Biphenol-Einheit zu verstehen, welche die folgende Struktur aufweist:

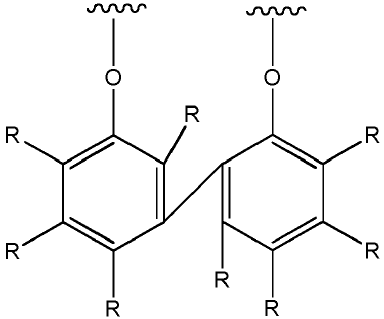

Die Nummerierung der C-Atome in den Ringen kann im IUPAC-Namen der Verbindung, je nach zusätzlichen Substituenten, zu einer anderen Positionsangabe als 2 und 3 führen.

In DE 10 2006 058 682 A1 werden Bisphosphite offenbart, welche unterschiedliche aber symmetrischen Flügel-Bausteine aufweisen, wie beispielsweis die Verbindung Ib auf Seite 8 der DE 10 2006 058 682 A1.

Die meisten im Stand der Technik bekannten Bisphosphite zeichnen sich dadurch aus, dass diese ein 2,2'-Biphenol als Zentralbaustein, also im Rückgrat, haben. Die Verwendung eines 2,3'-Biphenols als Zentralbaustein ist gänzlich unbekannt.

Der Erfindung lag die Aufgabe zugrunde, Bisphosphite bereitzustellen, welche gegenüber den in der Literatur bekannten Bisphosphiten eine neuartige Struktur aufweisen.

Die Aufgabe wird gelöst durch eine Verbindung nach Anspruch 1.

Verbindung, welche eine der drei allgemeinen Strukturen (**I**) bis **(III)** aufweist: 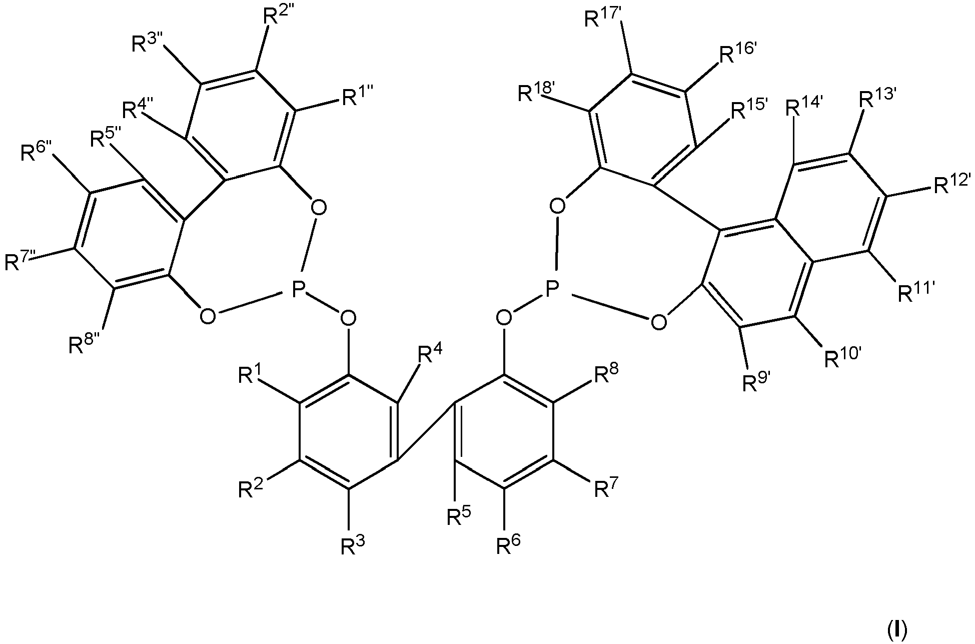 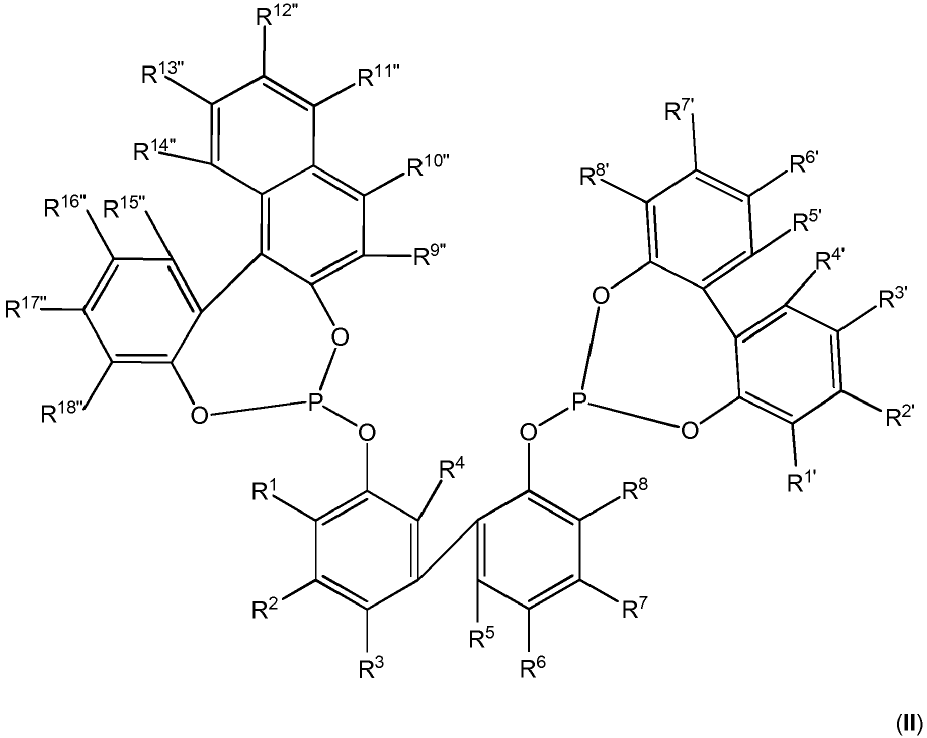 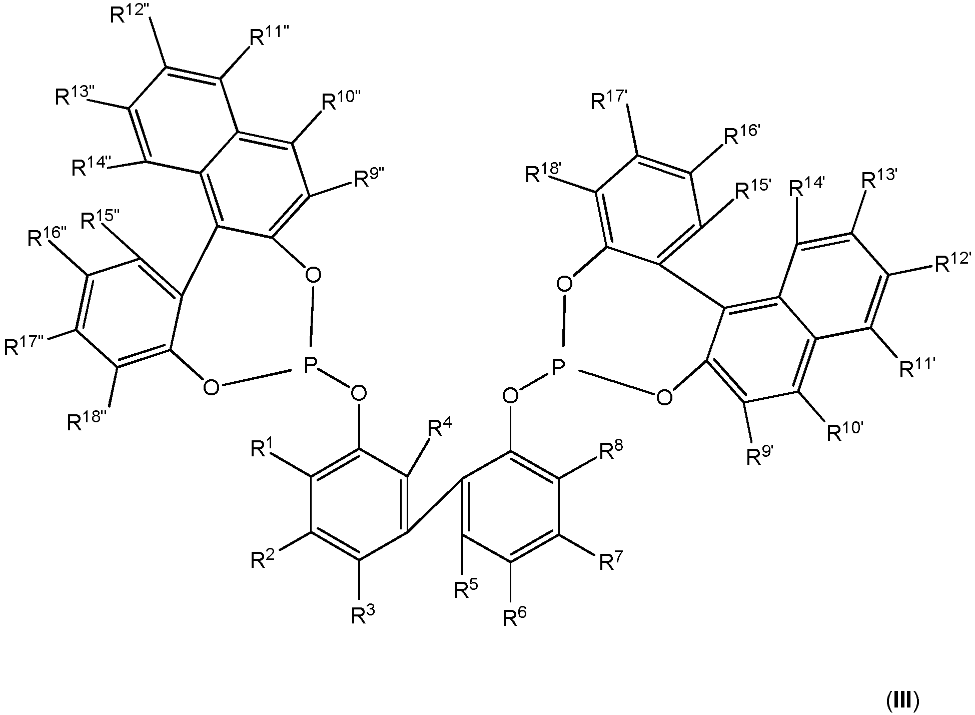 wobei
R¹, R², R³, R⁴, R⁵, R⁶, R⁷, R⁸ ausgewählt sind aus:
   -H, -(C₁-C₁₂)-Alkyl, -O-(C₁-C₁₂)-Alkyl, -O-(C₆-C₂₀)-Aryl, -(C₆-C₂₀)-Aryl, -S-Alkyl, -S-Aryl, Halogen, COO-(C₁-C₁₂)-Alkyl, CONH-(C₁-C₁₂)-Alkyl, , -CO-(C₁-C₁₂)-Alkyl, -CO-(C₆-C₂₀)-Aryl, -COOH, -OH, -SO₃H, -CN, -NH₂, -N[(C₁-C₁₂)-Alkyl]₂;
R¹', R²', R³', R⁴', R⁵', R⁶', R⁷', R⁸', R⁹', R¹⁰', R¹¹', R¹²', R¹³', R¹⁴', R¹⁵', R¹⁶', R¹⁷', R¹⁸' ausgewählt sind aus:
   -H, -(C₁-C₁₂)-Alkyl, -O-(C₁-C₁₂)-Alkyl, -O-(C₆-C₂₀)-Aryl, -(C₆-C₂₀)-Aryl, -S-Alkyl, -S-Aryl, Halogen, COO-(C₁-C₁₂)-Alkyl, CONH-(C₁-C₁₂)-Alkyl, -CO-(C₁-C₁₂)-Alkyl, -CO-(C₆-C₂₀)-Aryl, -COOH, -OH, - SO₃H, -NH₂, -N[(C₁-C₁₂)-Alkyl]₂;
R¹", R²", R³", R⁴", R⁵", R⁶", R⁷", R⁸", R⁹", R¹⁰", R¹¹", R¹²", R¹³", R¹⁴", R¹⁵", R¹⁶", R¹⁷", R¹⁸", ausgewählt sind aus:
   -H, -(C₁-C₁₂)-Alkyl, -O-(C₁-C₁₂)-Alkyl, -O-(C₆-C₂₀)-Aryl, -(C₆-C₂₀)-Aryl, -S-Alkyl, -S-Aryl, Halogen, COO-(C₁-C₁₂)-Alkyl, CONH-(C₁-C₁₂)-Alkyl, -CO-(C₁-C₁₂)-Alkyl, -CO-(C₆-C₂₀)-Aryl, -OH, -SO₃H,-NH₂, -N[(C₁-C₁₂)-Alkyl]₂;
wobei die genannten Alkyl- und Arylgruppen substituiert sein können.

-(C₁-C₁₂)-Alkyl und -O-(C₁-C₁₂)-Alkyl können jeweils unsubstituiert oder durch einen oder mehrere gleiche oder verschiedene Reste substituiert sein, die ausgewählt sind unter -(C₃-C₁₂)-Cycloalkyl, -(C₃-C₁₂)-Heterocycloalkyl, -(C₆-C₂₀)-Aryl, Fluor, Chlor, Cyano, Formyl, Acyl oder Alkoxycarbonyl.

-(C₆-C₂₀)-Aryl und -(C₆-C₂₀)-Aryl-(C₆-C₂₀)-Aryl- können jeweils unsubstituiert oder durch einen oder mehrere gleiche oder verschiedene Reste substituiert sein, die ausgewählt sind unter -H, -(C₁-C₁₂)-Alkyl, -O-(C₁-C₁₂)-Alkyl, -O-(C₆-C₂₀)-Aryl, -(C₆-C₂₀)-Aryl, -Halogen (wie Cl, F, Br, I), -COO-(C₁-C₁₂)-Alkyl, -CONH-(C₁-C₁₂)-Alkyl, -(C₆-C₂₀)-Aryl-CON[(C₁-C₁₂)-Alkyl]₂, -CO-(C₁-C₁₂)-Alkyl, -CO-(C₆-C₂₀)-Aryl, -COOH, -OH, -SO₃H, -SO₃Na, -NO₂, -CN, -NH₂, -N[(C₁-C₁₂)-Alkyl]₂.

Im Rahmen der Erfindung umfasst der Ausdruck -(C₁-C₁₂)-Alkyl geradkettige und verzweigte Alkylgruppen. Vorzugsweise handelt es sich dabei um unsubstituierte geradkettige oder verzweigte -(C₁-C₈)-Alkyl- und ganz bevorzugt -(C₁-C₆)-Alkylgruppen. Beispiele für -(C₁-C₁₂)-Alkylgruppen sind insbesondere Methyl, Ethyl, Propyl, Isopropyl, n-Butyl, iso-Butyl, sec.-Butyl, tert.-Butyl, n-Pentyl, 2-Pentyl, 2-Methylbutyl-, 3-Methylbutyl-, 1,2-Dimethylpropyl-, 1,1-Dimethylpropyl, 2,2-Dimethylpropyl-, 1-Ethylpropyl-, n-Hexyl-, 2-Hexyl-, 2-Methylpentyl-, 3-Methylpentyl-, 4-Methylpentyl-, 1,1-Dimethylbutyl-, 1,2-Diemthylbutyl-, 2,2-Dimethylbutyl-, 1,3-Dimethylbutyl-, 2,3-Dimethylbutyl-, 3,3-Dimethylbutyl-, 1,1,2-Trimethylpropyl-, 1,2,2-Trimethylpropyl-, 1-Ethylbutyl-, 1-Ethyl-2-methylpropyl-, n-Heptyl-, 2-Heptyl-, 3-Heptyl-, 2-Ethylpentyl-, 1-Propylbutyl-, n-Octyl-, 2-Ethylhexyl-, 2-Propylheptyl-, Nonyl-, Decyl.

Die Erläuterungen zum Ausdruck -(C₁-C₁₂)-Alkyl gelten auch für die Alkylgruppen in -O-(C₁-C₁₂)-Alkyl, also in -(C₁-C₁₂)-Alkoxy. Vorzugsweise handelt es sich dabei um unsubstituierte geradkettige oder verzweigte -(C₁-C₆)-Alkoxygruppen.

Substituierte -(C₁-C₁₂)-Alkylgruppen und substituierte -(C₁-C₁₂)-Alkoxygruppen können in Abhängigkeit von ihrer Kettenlänge, einen oder mehrere Substituenten aufweisen. Die Substituenten sind vorzugsweise unabhängig voneinander ausgewählt unter -(C₃-C₁₂)-Cycloalkyl, -(C₃-C₁₂)-Heterocycloalkyl, -(C₆-C₂₀)-Aryl, Fluor, Chlor, Cyano, Formyl, Acyl oder Alkoxycarbonyl.

Der Ausdruck -(C₃-C₁₂)-Cycloalkyl umfasst im Sinne der vorliegenden Erfindung mono-, bi- oder tricyclische Kohlenwasserstoffreste mit 3 bis 12, insbesondere 5 bis 12 Kohlenstoffatomen. Dazu zählen Cyclopropyl-, Cyclobutyl-, Cyclopentyl-, Cyclohexyl-, Cycloheptyl-, Cyclooctyl-, Cyclododecyl-, Cyclopentadecyl-, Norbonyl- oder Adamantyl.
Ein Beispiel für ein substituiertes Cycloalkyl wäre Menthyl.

Der Ausdruck -(C₃-C₁₂)-Heterocycloalkylgruppen umfasst im Sinne der vorliegenden Erfindung nichtaromatische, gesättigte oder teilweise ungesättigte cycloaliphatische Gruppen mit 3 bis 12, insbesondere 5 bis 12, Kohlenstoffatomen. Die -(C₃-C₁₂)-Heterocycloalkylgruppen weisen vorzugsweise 3 bis 8, besonders bevorzugt 5 oder 6, Ringatome auf. In den Heterocycloalkylgruppen sind im Unterschied zu den Cycloalkylgruppen 1, 2, 3 oder 4 der Ringkohlenstoffatome durch Heteroatome oder heteroatomhaltige Gruppen ersetzt. Die Heteroatome oder die heteroatomhaltige Gruppen sind vorzugsweise ausgewählt unter -O-, -S-, -N-, -N(=O)-, -C(=O)- oder -S(=O)-. Beispiele für -(C₃-C₁₂)-Heterocycloalkylgruppen Tetrahydrothiophenyl, Tetrhydrofuryl, Tetrahydropyranyl und Dioxanyl.

Der Ausdruck -(C₆-C₂₀)-Aryl und -(C₆-C₂₀)-Aryl-(C₆-C₂₀)-Aryl- umfasst im Sinne der vorliegenden Erfindung mono- oder polycyclische aromatische Kohlenwasserstoffreste. Diese weisen 6 bis 20 Ringatome, besonders bevorzugt 6 bis 14 Ringatome, insbesondere 6 bis 10 Ringatome, auf. Aryl steht vorzugsweise für -(C₆-C₁₀)-Aryl und -(C₆-C₁₀)-Aryl-(C₆-C₁₀)-Aryl-. Aryl steht insbesondere für Phenyl, Naphthyl, Indenyl, Fluorenyl, Anthracenyl, Phenanthrenyl, Naphthacenyl, Chrysenyl, Pyrenyl, Coronenyl. Insbesondere steht Aryl für Phenyl, Naphthyl und Antracenyl.

Substituierte -(C₆-C₂₀)-Arylgruppen und -(C₆-C₂₀)-Aryl-(C₆-C₂₀)-Arylgruppen können, in Abhängigkeit von der Ringgröße, einen oder mehrere (z.B. 1, 2, 3, 4 oder 5) Substituenten aufweisen. Diese Substituenten sind vorzugsweise unabhängig voneinander ausgewählt unter - H, -(C₁-C₁₂)-Alkyl, -O-(C₁-C₁₂)-Alkyl, -O-(C₆-C₂₀)-Aryl, -(C₆-C₂₀)-Aryl, -Halogen (wie Cl, F, Br, I),-COO-(C₁-C₁₂)-Alkyl, -CONH-(C₁-C₁₂)-Alkyl, -(C₆-C₂₀)-Aryl-CON[(C₁-C₁₂)-Alkyl]₂, -CO-(C₁-C₁₂)-Alkyl, -CO-(C₆-C₂₀)-Aryl, -COOH, -OH, -SO₃H; -SO₃Na, -NO₂, -CN, -NH₂, -N[(C₁-C₁₂)-Alkyl]₂. Substituierte -(C₆-C₂₀)-Arylgruppen und -(C₆-C₂₀)-Aryl-(C₆-C₂₀)-Arylgruppen sind vorzugsweise substituierte -(C₆-C₁₀)-Arylgruppen und -(C₆-C₁₀)-Aryl-(C₆-C₁₀)-Arylgruppen, insbesondere substituiertes Phenyl oder substituiertes Naphthyl oder substituiertes Anthracenyl. Substituierte -(C₆-C₂₀)-Arylgruppen tragen vorzugsweise eine oder mehrere z.B. 1, 2, 3, 4 oder 5 Substituenten, ausgewählt unter -(C₁-C₁₂)-Alkylgruppen, -(C₁-C₁₂)-Alkoxygruppen.

In einer Ausführungsform sind R¹, R², R³, R⁴, R⁵, R⁶, R¹, R⁸ ausgewählt aus:
-H, -(C₁-C₁₂)-Alkyl, -O-(C₁-C₁₂)-Alkyl, -O-(C₆-C₂₀)-Aryl, -S-Alkyl, -S-Aryl.

In einer Ausführungsform sind R¹', R²', R³', R⁴', R⁵', R⁶', R⁷', R⁸' ausgewählt aus:
-H, -(C₁-C₁₂)-Alkyl, -O-(C₁-C₁₂)-Alkyl, -O-(C₆-C₂₀)-Aryl, -S-Alkyl, -S-Aryl.

In einer Ausführungsform sind R¹", R²", R³", R⁴", R⁵", R⁶", R⁷", R⁸" ausgewählt aus:
-H, -(C₁-C₁₂)-Alkyl, -O-(C₁-C₁₂)-Alkyl, -O-(C₆-C₂₀)-Aryl, -S-Alkyl, -S-Aryl.

In einer Ausführungsform sind R⁹', R¹⁰' R¹¹', R¹²', R¹³', R¹⁴', R¹⁵', R¹⁶', R¹⁷', R¹⁸', ausgewählt aus:
-H, -(C₁-C₁₂)-Alkyl, -O-(C₁-C₁₂)-Alkyl, -O-(C₆-C₂₀)-Aryl, -S-Alkyl, -S-Aryl.

In einer Ausführungsform sind R⁹", R¹⁰", R¹¹", R¹²", R¹³", R¹⁴", R¹⁵", R¹⁶", R¹⁷", R¹⁸" ausgewählt aus:
-H, -(C₁-C₁₂)-Alkyl, -O-(C₁-C₁₂)-Alkyl, -O-(C₆-C₂₀)-Aryl, -S-Alkyl, -S-Aryl.

In einer Ausführungsform sind R¹, R², R³, R⁴, R⁵, R⁶, R⁷, R⁸ ausgewählt aus:
-H, -(C₁-C₁₂)-Alkyl, -O-(C₁-C₁₂)-Alkyl, -O-(C₆-C₂₀)-Aryl.

In einer Ausführungsform sind R¹', R²', R³', R⁴', R⁵', R⁶', R⁷', R⁸' ausgewählt aus:
-H, -(C₁-C₁₂)-Alkyl, -O-(C₁-C₁₂)-Alkyl, -O-(C₆-C₂₀)-Aryl.

In einer Ausführungsform sind R¹", R²", R³", R⁴", R⁵", R⁶", R⁷", R⁸" ausgewählt aus:
-H, -(C₁-C₁₂)-Alkyl, -O-(C₁-C₁₂)-Alkyl, -O-(C₆-C₂₀)-Aryl.

In einer Ausführungsform sind R⁹', R¹⁰' R¹¹', R¹²', R¹³', R¹⁴', R¹⁵', R¹⁶', R¹⁷', R¹⁸', ausgewählt aus:
-H, -(C₁-C₁₂)-Alkyl, -O-(C₁-C₁₂)-Alkyl, -O-(C₆-C₂₀)-Aryl.

In einer Ausführungsform sind R⁹", R¹⁰", R¹¹", R¹²", R¹³", R¹⁴", R¹⁵", R¹⁶", R¹⁷", R¹⁸" ausgewählt aus:
-H, -(C₁-C₁₂)-Alkyl, -O-(C₁-C₁₂)-Alkyl, -O-(C₆-C₂₀)-Aryl.

In einer Ausführungsform weist die Verbindung die allgemeine Struktur (I) auf.

In einer Ausführungsform weist die Verbindung die allgemeine Struktur **(II)** auf.

In einer Ausführungsform weist die Verbindung die allgemeine Struktur **(III)** auf.

Im Folgenden wird die Erfindung anhand von Ausführungsbeispielen und einer Figur näher erläutert.

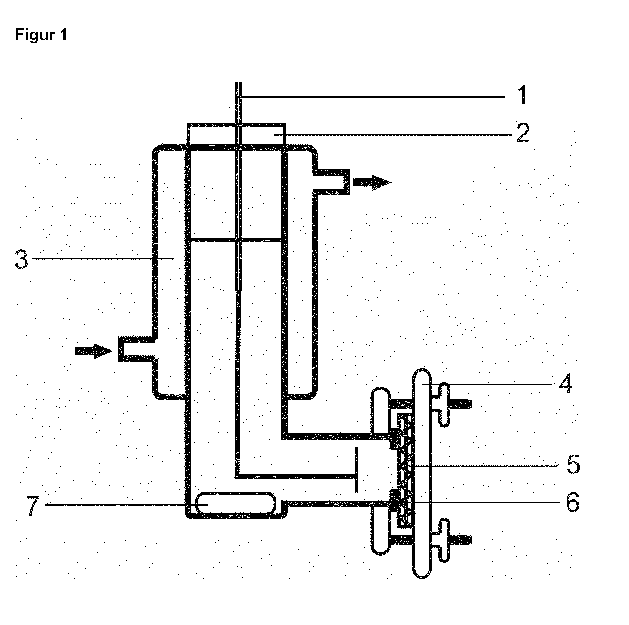

Die Figur 1 zeigt eine Reaktionsapparatur, in welcher die Kupplungsreaktion zu den entsprechenden unsymmetrischen Biarylen durchgeführt werden kann. Die Apparatur umfasst eine Nickelkathode (1) und eine Anode aus Bor-dotiertem Diamant (BDD) auf Silizium (5). Die Apparatur kann mit Hilfe des Kühlmantels (3) gekühlt werden. Die Pfeile deuten hierbei die Durchflussrichtung des Kühlwassers an. Der Reaktionsraum ist mit einem Teflonstopfen (2) verschlossen. Das Reaktionsgemisch wird durch ein Magnetrührstäbchen (7) durchmischt. Auf der anodischen Seite wird die Apparatur durch Schraubzwingen (4) und Dichtungen (6) verschlossen.

### Analytik

### Chromatographie

Die präparativen flüssigkeitschromatographischen Trennungen via "Flashchromatographie" wurden mit einem Maximaldruck von 1.6 bar an Kieselgel 60 M (0.040-0.063 mm) der Firma Macherey-Nagel GmbH & Co, Düren durchgeführt. Die Trennungen ohne Druckbeaufschlagung wurden an Kieselgel Geduran Si 60 (0.063-0.200 mm) der Firma Merck KGaA, Darmstadt durchgeführt. Die als Eluentien verwendeten Lösungsmittel (Essigsäureethylester (technisch), Cyclohexan (technisch)) wurden zuvor destillativ am Rotationsverdampfer gereinigt.
Zur Dünnschichtchromatographie (DC) wurden PSC-Fertigplatten Kieselgel 60 F254 der Firma Merck KGaA, Darmstadt verwendet. Die Rf-Werte sind in Abhängigkeit vom verwendeten Laufmittelgemisch angegeben. Zur Anfärbung der DC-Platten wurde eine Cer-Molybdatophosphorsäure-Lösung als Tauchreagenz verwendet. Cer-Molybdatophosphorsäure-Reagenz: 5.6 g Molybdatophosphorsäure, 2.2 g Cer(IV)-sulfat-Tetrahydrat und 13.3g konzentrierte Schwefelsäure auf 200 mL Wasser.

### Gaschromatographie (GC/GCMS)

Die gaschromatographischen Untersuchungen (GC) von Produktgemischen und Reinsubstanzen erfolgte mit Hilfe des Gaschromatographen GC-2010 der Firma Shimadzu, Japan. Es wird an einer Quarzkapillarsäule HP-5 der Firma Agilent Technologies, USA (Länge: 30 m; Innendurchmesser: 0.25 mm; Filmdicke der kovalent gebundenen stationären Phase: 0.25 µm; Trägergas: Wasserstoff; Injektortemperatur: 250 °C; Detektortemperatur: 310 °C; Programm: Methode "hart": 50 °C Starttemperatur für 1 min, Heizrate: 15 °C/min, 290 °C Endtemperatur für 8 min) gemessen. Gaschromatographische Massenspektren (GCMS) von Produktgemischen und Reinsubstanzen wurden mit Hilfe des Gaschromatographen GC-2010 kombiniert mit dem Massendetektor GCMS-QP2010 der Firma Shimadzu, Japan aufgenommen. Es wird an einer Quarzkapillarsäule HP-1 der Firma Agilent Technologies, USA (Länge: 30 m; Innendurchmesser: 0.25 mm; Filmdicke der kovalent gebundenen stationären Phase: 0.25 µm; Trägergas: Wasserstoff; Injektortemperatur: 250 °C; Detektortemperatur: 310 °C; Programm: Methode "hart": 50 °C Starttemperatur für 1 min, Heizrate: 15 °C/min, 290 °C Endtemperatur für 8 min; GCMS: Temperatur der lonenquelle: 200 °C) gemessen.

### Schmelzpunkte

Schmelzpunkte wurden mit Hilfe des Schmelzpunktbestimmungsgerätes SG 2000 der Firma HW5, Mainz gemessen und sind unkorrigiert.

### Elementaranalyse

Die Elementaranalysen wurden in der analytischen Abteilung des Institutes für Organische Chemie der Johannes Gutenberg-Universität Mainz an einem Vario EL Cube der Firma Foss-Heraeus, Haunau angefertigt.

### Massenspektrometrie

Alle Elektrosprayionisation-Messungen (ESI+) wurden an einem QTof Ultima 3 der Firma Waters Micromasses, Milford, Massachusetts durchgeführt. EI-Massenspektren sowie die hochaufgelösten EI-Spektren wurden an einem Gerät des Typs MAT 95 XL Sektorfeldgerät der Firma ThermoFinnigan, Bremen, gemessen.

### NMR-Spektroskopie

Die NMR-spektroskopischen Untersuchungen wurden an Multikernresonanzspektrometern des Typs AC 300 oder AV II 400 der Firma Bruker, Analytische Messtechnik, Karlsruhe, durchgeführt. Als Lösungsmittel wurde CDCl3 verwendet. Die 1H- und 13C-Spektren wurden gemäß dem Restgehalt an nicht deuteriertem Lösungsmittel nach der NMR Solvent Data Chart der Fa. Cambridge Isotopes Laboratories, USA, kalibriert. Die Zuordnung der 1H- und 13C-Signale erfolgte teilweise mit Hilfe von H,H-COSY, H,H-NOESY, H,C-HSQC und H,C-HMBC-Spektren. Die chemischen Verschiebungen sind als δ-Werte in ppm angegeben. Für die Multiplizitäten der NMR-Signale wurden folgende Abkürzungen verwendet: s (Singulett), bs (breites Singulett), d (Dublett), t (Triplett), q (Quartett), m (Multiplett), dd (Dublett von Dublett), dt (Dublett von Triplett), tq (Triplett von Quartett). Alle Kopplungskonstanten J wurden mit der Anzahl der eingeschlossenen Bindungen in Hertz (Hz) angegeben. Die bei der Signalzuordnung angegebene Nummerierung entspricht der in den Formelschemata angegebenen Bezifferung, die nicht mit der IUPAC-Nomenklatur übereinstimmen muss.

### Allgemeine Arbeitsvorschriften

Alle nachfolgenden Präparationen wurden mit Standard-Schlenk-Technik unter Schutzgas durchgeführt. Die Lösungsmittel wurden vor Gebrauch über geeigneten Trocknungsmitteln getrocknet (Purification of Laboratory Chemicals, W. L. F. Armarego (Autor), Christina Chai (Autor), Butterworth Heinemann (Elsevier), 6. Auflage, Oxford 2009).
Phosphortrichlorid (Aldrich) wurde vor dem Einsatz unter Argon destilliert. Alle präparativen Arbeiten erfolgten in ausgeheizten Gefäßen. Die Charakterisierung der Produkte erfolgte mittels NMR-Spektroskopie. Chemische Verschiebungen (δ) werden in ppm angegeben. Die Referenzierung der ³¹P-NMR-Signale erfolgte gemäß: SR_{31P} = SR_{1H} * (BF_{31P} /BF_{1H}) = SR_{1H} * 0,4048. (Robin K. Harris, Edwin D. Becker, Sonia M. Cabral de Menezes, Robin Goodfellow, and Pierre Granger, Pure Appl. Chem., 2001, 73, 1795-1818; Robin K. Harris, Edwin D. Becker, Sonia M. Cabral de Menezes, Pierre Granger, Roy E. Hoffman and Kurt W. Zilm, Pure Appl. Chem., 2008, 80, 59-84).

### Synthese der unsymmetrischen Biaryle

Die unsymmetrischen Biaryle wurden durch ein elektrochemisches Verfahren durch Kupplung von zwei Phenolen bzw. von einem Naphthol mit einem Phenol, welche sich in ihrem Oxidationspotential unterscheiden, hergestellt. Siehe hierzu auch B. Elsler, D. Schollmeyer, K. M. Dyballa, R. Franke, S. R. Waldvogel, "Metall- und reagensfreie hochselektive anodische Kreuzkupplung von Phenolen", Angew. Chem., 2014, DOI: 10.1002/ange.201400627

### Allgemeine Arbeitsvorschrift:

Die Kupplungsreaktion wurde in einer Apparatur durchgeführt, wie sie in Figur 1 dargestellt ist. 5 mmol des ersten Phenols mit einem Oxidationspotential *E_{Ox}1* werden mit 15 mmol des zweiten Phenols mit einem Oxidationspotential *E_{Ox}2* in den in der nachfolgenden Tabelle 1 angegebenen Mengen in 1,1,1,3,3,3-Hexafluorisopropanol (HFIP) und MeOH oder in Ameisensäure und MeOH gelöst. Die Elektrolyse erfolgt galvanostatisch. Der Außenmantel der Elektrolysezelle wird über einen Thermostaten auf etwa 10 °C temperiert, während die Reaktionsmischung gerührt und auf 50 °C mit Hilfe eines Sandbades erhitzt wird. Nach Ende der Elektrolyse wird der Zellinhalt mit Toluol in einen 50 mL Rundhalskolben überführt und das Lösungsmittel unter vermindertem Druck am Rotationsverdampfer bei 50 °C, 200-70 mbar entfernt. Nicht umgesetztes Edukt wird mittels Kurzwegdestillation zurückerhalten (100 °C, 10⁻³ mbar).

**Elektrodenmaterial**

| | |
|---|---|
| Anode: | Bor-dotierter Diamant (BDD) auf Si |
| Kathode: | Ni-Netz |

**Elektrolysebedingungen:**

| | |
|---|---|
| Temperatur [T]: | 50 °C |
| Stromstärke [I]: | 15 mA |
| Stromdichte [j]: | 2.8 mA/cm² |
| Ladungsmenge [Q]: | 2 F/mol Unterschusskomponente |
| Klemmspannung [Uₘₐₓ]: | 3-5 V |

Die Synthese der Biaryle erfolgte gemäß der oben beschriebenen allgemeinen Arbeitsvorschrift, und in einer Reaktionsapparatur, wie sie in Figur 1 dargestellt ist.

Bei den Strukturen (**I**) und **(II)** besteht je ein Flügelbaustein aus einer 2,2'-Biphenol-Einheit.

Die Synthese von symmetrischen 2,2'-Biphenol-Einheiten ist dem Fachmann hinreichend bekannt und kann der gängigen Fachliteratur entnommen werden.

### 2,2'-Dihydroxy-4',5-dimethyl-5'-(methylethyl)-3-methoxybiphenyl

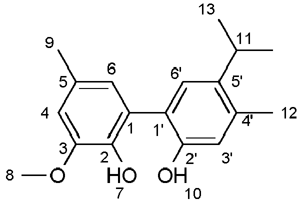

Es wurden 0.69 g (5 mmol, 1.0 Äquiv.) 4-Methylguajacol und 2.28 g (15 mmol, 3.0 Äquiv.) 3-Methyl-4-(methylethyl)phenol in 33 mL HFIP gelöst, 0.68 g Methyltriethylammoniummethylsulfat (MTES) zugegeben und der Elektrolyt in die Elektrolysezelle überführt. Das Lösungsmittel sowie nicht umgesetzte Eduktmengen werden nach der Elektrolyse unter vermindertem Druck entfernt, das Rohprodukt an Kieselgel 60 als "Flashchromatographie" im Laufmittel 9:1 (Cyclohexan:Essigsäureethylester) aufgereinigt und das Produkt als farbloser Feststoff erhalten.
Ausbeute: 716 mg (50%, 2.5 mmol)

GC (Methode *hart,* HP-5): t_{R}= 14.87 min
R_{f}(CH:EE= 4:1)= 0.43
mₚ= 126.8°C (aus CH umkristallisiert)
¹H-NMR (600 MHz, DMSO) δ= 1.17-1.12 (m, 6H, 13-H), 2.24 (m, 6H, 9-H/12-H), 3.01 (dt, 1H, 11-H), 3.79 (s, 3H, 8-H), 6.55 (s, 1 H, 6-H), 6.66 (d, 1 H, 6'-H), 6.73 (d, 1 H, 4-H), 6.96 (s, 1 H, 3'-H), 8.16 (s, 1H, 7-H), 8.84 (s, 1H, 10-H);
Kopplungen: ⁴J_{4-H, 6-H}= 2.2 Hz, ⁴J_{6'-H, 11-H}= 2.9 Hz, ³J_{11-H, 13-H}= 6.8 Hz;
¹³C-NMR (151 MHz, DMSO) δ= 18.73, 20.80 (C-9/C-12), 23.54 (C-13), 28.10 (C-11), 55.78 (C-8), 111.23 (C-4), 117.34 (C-6'), 123.42 (C-1'), 123.49 (C-6), 126.43 (C-1), 127.36 (C-5), 127.49 (C-3'), 134.40 (C-5'), 136.62 (C-4'), 141.12 (C-2), 147.65 (C-3), 151.69 (C-2').
HRMS für C₁₈H₂₂O₃ (ESI+) [M+Na⁺]: ber: 309.1467, gef.: 309.1457
MS (EI, GCMS): m/z(%): 286 (50) [M]^{+.}, 271 (100) [M-CH₃˙]⁺, 244 (22) [M-C₃H₆˙]⁺. Elementaranalyse für C₁₈H₂₂O_{3:} ber: C: 75.50%, H: 7.74%, gef.: C: 75.01%, H: 7.70%.

### 2,2'-Dihydroxy-5,5'-dimethyl-3-methoxybiphenyl

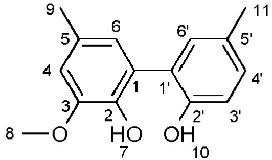

Es wurden 1.66 g (12 mmol, 1.0 Äquiv.) 4-Methylguajacol und 3.91 g (36 mmol, 3.0 Äquiv.) 4-Methylphenol in 65 mL HFIP und 14 mL MeOH gelöst, 1.63 g Methyltriethylammoniummethylsulfat (MTES) zugegeben und der Elektrolyt in die Elektrolysezelle überführt. Das Lösungsmittel sowie nicht umgesetzte Eduktmengen werden nach der Elektrolyse unter vermindertem Druck entfernt, das Rohprodukt an Kieselgel 60 als "Flashchromatographie" im Laufmittel 4:1 (Cyclohexan:Essigsäureethylester) aufgereinigt und das Produkt als farbloser Feststoff erhalten.
Ausbeute: 440 mg (36%, 1.8 mmol)

GC (Methode *hart,* HP-5): t_{R}= 13.56 min
R_{f}(CH:EE= 4:1)= 0.38
mₚ= 162.0 °C (aus CH umkristallisiert)
¹H-NMR (400 MHz, DMSO) δ= 2.18 (s, 3H, 9-H/11-H), 2.21 (s, 3H, 9-H/11-H), 3.76 (s, 3H, 8-H), 6.53 (s, 1H, 6-H), 6.71 (s, 1H, 4-H), 6.75 (d, 1H, 3'-H), 6.86-6.94 (m, 2H, 4'-H/6'-H), 8.53 (bs, 1H, 7-H/12-H);
Kopplungen: ³J_{3'-H, 4'-H}= 8.4 Hz;
¹³C-NMR (101 MHz, DMSO) δ= 20.21, 20.77 (C-9/C-11), 55.79 (C-8), 111.36 (C-4), 115.69 (C-3'), 123.50 (C-6), 125.72 (C-1'), 126.16 (C-1), 127.20 (C-5), 127.30 (C-5'), 128.50 (C-6'), 131.83 (C-4'), 141.20 (C-2), 147.61 (C-3), 152.11 (C-2').
HRMS für C₁₅H₁₆O₃ (ESI+) [M+Na⁺]: ber: 267.0997, gef.: 267.0999
MS (EI, GCMS): m/z(%): 244 (100) [M]⁺˙, 229 (64) [M-CH₃**]**˙⁺.
Elementaranalyse für C₁₅H₁₆O_{3:} ber: C: 73.75%, H: 6.60%, gef.: C: 73.81%, H: 6.54%.

### 2,2'-Dihydroxy-3-methoxy-3',5,5'-trimethyl-biphenyl

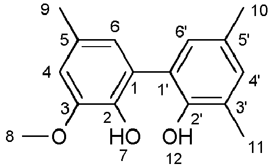

Es wurden 0.70 g (6 mmol, 1.0 Äquiv.) 4-Methylguajacol und 2.08 g (17 mmol, 3.0 Äquiv.) 2,4-Dimethylphenol in 27 mL HFIP und 6 mL MeOH gelöst, 0.68 g Methyltriethylammoniummethylsulfat (MTES) zugegeben und der Elektrolyt in die Elektrolysezelle überführt. Das Lösungsmittel sowie nicht umgesetzte Eduktmengen werden nach der Elektrolyse unter vermindertem Druck entfernt, das Rohprodukt an Kieselgel 60 als "Flashchromatographie" im Laufmittel 9:1 (Cyclohexan:Essigsäureethylester) aufgereinigt und das Produkt als leichtgelber Feststoff erhalten.
Ausbeute: 663 mg (45%, 2.5 mmol)

GC (Methode *hart,* HP-5): t_{R}= 13.97 min
R_{f}(CH:EE= 4:1)= 0.29
mₚ= 119.7 °C (aus DCM/CH umkristallisiert)
¹H-NMR (400 MHz, CDCl₃) δ= 2.34 (s, 3H, 10-H), 2.35 (s, 3H, 11-H), 2.38 (s, 3H, 9-H), 3.94 (s, 3H, 8-H), 6.16 (s, 1H, 12-H), 6.20 (s, 1H, 7-H), 6.76 (d, 1H, 4-H), 6.78 (d, 1H, 6-H), 6.98 (d, 1H, 6'-H), 7.03 (d, 1 H, 4'-H);
Kopplungen: ⁴J_{4-H, 6-H}= 1.7 Hz, ⁴J_{4'-H, 6'-H}= 2.1 Hz;
¹³C-NMR (101 MHz, CDCl₃) δ= 16.51 (C-9), 20.54 (C-10), 21.20 (C-11), 56.12 (C-8), 110.92 (C-4), 123.95 (C-6), 124.13 (C-1), 124.64 (C-1'), 126.18 (C-3'), 128.82 (C-6'), 129.59 (C-5'), 130.40 (C-5), 131.40 (C-4'), 139.46 (C-2), 146.35 (C-3), 149.42 (C-2').
HRMS für C₁₈H₁₆O₃ (ESI+) [M+Na⁺]: ber: 281.1154, gef.: 281.1152
MS (EI, GCMS): m/z(%): 242 (100) [M]⁺˙, 227 (38) [M-CH₃˙]⁺.
Elementaranalyse für C₁₆H₁₈O_{3:} ber: C: 68.31%, H: 6.45%, gef.: C: 68.29%, H: 6.40%.

### 2,2'-Dihydroxy-3-methoxy-5-methyl-4'-(dimethylethyl)biphenyl

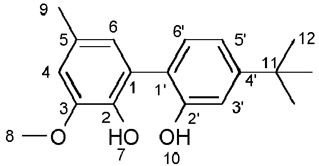

Es wurden 0.69 g (5 mmol, 1.0 Äquiv.) 4-Methylguajacol und 2.25 g (15 mmol, 3.0 Äquiv.) 3-tert-Butylphenol in 33 mL HFIP gelöst, 0.68 g Methyltriethylammoniummethylsulfat (MTES) zugegeben und der Elektrolyt in die Elektrolysezelle überführt. Das Lösungsmittel sowie nicht umgesetzte Eduktmengen werden nach der Elektrolyse unter vermindertem Druck entfernt, das Rohprodukt an Kieselgel 60 als "Flashchromatographie" im Laufmittel 4:1 (Cyclohexan:Essigsäureethylester) aufgereinigt und das Produkt als farbloser Feststoff erhalten.
Ausbeute: 808 mg (63%, 3.1 mmol)

GC (Methode *hart,* HP-5): t_{R}= 13.97 min
R_{f}(CH:EE= 4:1)= 0.29
mₚ= 160.3 °C (aus DCM/CH umkristallisiert)
¹H-NMR (400 MHz, CDCl₃) δ= 1.37 (s, 9H, 12-H), 2.36 (s, 3H, 9-H), 3.94 (s, 3H, 8-H), 6.25 (s, 1 H, 7-H), 6.48 (s, 1 H, 10-H), 6.75 (d, 1 H, 6-H), 6.79 (d, 1 H, 4-H), 7.08 (dd, 1 H, 5'-H), 7.12 (d, 1 H, 3'-H), 7.27 (d, 1 H, 6'-H);
Kopplungen: ⁴J_{4-H, 6-H}= 1.7 Hz; ³J_{5'-H, 6'-H}= 8.0 Hz, ⁴J_{3'-H, 5'-H}= 1.7 Hz;
¹³C-NMR (101 MHz, CDCl₃) δ= 21.24 (C-9), 31.31 (C-12), 34.58 (C-11), 56.15 (C-8), 110.79 (C-4), 114.94 (C-3'), 118.30 (C-5'), 122.37 (C-1'), 123.88 (C-1), 123.94 (C-6), 130.45 (C-6'), 130.53 (C-4'), 139.24 (C-5), 146.32 (C-3), 152.91 (C-2'), 153.13 (C-2).
HRMS für C₁₅H₁₆O₄ (ESI+) [M+Na⁺]: ber: 309.1467, gef.: 309.1466
MS (EI, GCMS): m/z(%): 242 (100) [M]⁺˙, 227 (38) [M-CH₃˙]⁺.
Elementaranalyse für C₁₈H₂₂O_{3:} ber: 75.50%, H: 7.74%, gef.: C: 75.41%, H: 7.72%.

### 2,2'-Dihydroxy-4',5-dimethy-3-methoxylbiphenyl

Es wurden 0.70 g (5 mmol, 1.0 Äquiv.) 4-Methylguajacol und 1.65 g (15 mmol, 3.0 Äquiv.) 3-Methylphenol in 33 mL HFIP gelöst, 0.68 g Methyltriethylammoniummethylsulfat (MTES) zugegeben und der Elektrolyt in die Elektrolysezelle überführt. Das Lösungsmittel sowie nicht umgesetzte Eduktmengen werden nach der Elektrolyse unter vermindertem Druck entfernt, das Rohprodukt an Kieselgel 60 als "Flashchromatographie" im Laufmittel 4:1 (Cyclohexan:Essigsäureethylester) aufgereinigt und zwei Kreuzkupplungsprodukte als farblose Feststoffe erhalten.

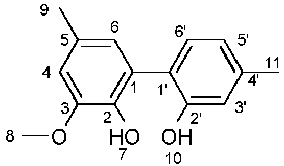

Ausbeute: 266 mg (21%, 1.1 mmol)
GC (Methode *hart,* HP-5): t_{R}= 13.72 min
R_{f}(CH:EE= 4:1)= 0.25
mₚ= 136.2 °C (aus DCM/CH umkristallisiert)
¹H-NMR (400 MHz, CDCl₃) δ= 2.35 (s, 3H, 9-H/11-H), 2.37 (s, 3H, 9-H/11-H), 3.94 (s, 3H, 8-H), 6.17 (s, 1 H, 10-H), 6.35 (s, 1 H, 2-H), 6.74 (d, 1 H, 4-H), 6.76 (s, 1 H, 6-H), 6.88-6.83 (m, 1 H, 5'-H), 6.90 (d, 1 H, 3'-H), 7.21 (d, 1 H, 6'-H);
Kopplungen: ⁴J_{4-H, 6-H}= 1.8 Hz, ³J_{5'-H, 6'-H}= 7.7 Hz, ⁴J_{3'-H, 5'-H}= 1.5 Hz;
¹³C-NMR (101 MHz, CDCl₃) δ= 21.11, 21.20 (C-9/C-11), 56.13 (C-8), 110.81 (C-4), 118.25 (C-3'), 121.97 (C-5'), 122.39 (C-1), 123.77 (C-1'), 123.85 (C-6), 130.50 (C-5), 130.68 (C-6'), 139.30 (C-4'), 139.54 (C-2), 146.31 (C-3), 153.33 (C-2').
HRMS für C₁₅H₁₆O₃ (ESI+) [M+Na⁺]: ber: 267.0997, gef.: 267.1006
MS (EI, GCMS): m/z(%): 244 (100) [M]⁺˙, 229 (18) [M-CH₃˙]⁺,.
Elementaranalyse für C₁₅H₁₆O_{3:} ber: C: 73.75%, H: 6.60%, gef.: C: 73.70%, H: 6.68%.

### 2,2'-Dihydroxy-3-methoxy-4'-5,5'-trimethylbiphenyl

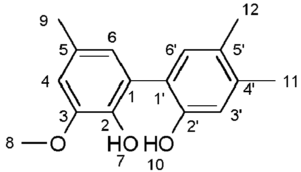

Es wurden 0.69 g (5 mmol, 1.0 Äquiv.) 4-Methylguajacol und 1.83 g (15 mmol, 3.0 Äquiv.) 3,4-Dimethylphenol in 27 mL HFIP und 6 mL MeOH gelöst, 0.68 g Methyltriethylammoniummethylsulfat (MTES) zugegeben und der Elektrolyt in die Elektrolysezelle überführt. Das Lösungsmittel sowie nicht umgesetzte Eduktmengen werden nach der Elektrolyse unter vermindertem Druck entfernt, das Rohprodukt an Kieselgel 60 als "Flashchromatographie" im Laufmittel 9:1 (Cyclohexan:Essigsäureethylester) aufgereinigt und das Produkt als farbloser Feststoff erhalten.
Ausbeute: 688 mg (52%, 2.6 mmol)

GC (Methode *hart,* HP-5): t_{R}= 14.52 min
R_{f}(CH:EE= 4:1)= 0.29
mₚ= 152.3 °C (aus DCM/CH umkristallisiert)
¹H-NMR (400 MHz, CDCl₃) δ= 12.25 (s, 3H, 11-H), 2.28 (s, 3H, 12-H), 2.36 (s, 3H, 9-H), 3.93 (s, 3H, 8-H), 6.19 (s, 1 H, 7-H), 6.25 (s, 1 H, 10-H), 6.73 (d, 1 H, 4-H), 6.76 (s, 1 H, 6-H), 6.88 (s, 1 H, 3'-H), 7.08 (s, 1 H, 6'-H);
Kopplungen: ⁴J_{4-H, 6-H}= 1.7 Hz;
¹³C-NMR (101 MHz, CDCl₃) δ= 18.89 (C-11), 19.60 (C-12), 21.24 (C-9), 56.14 (C-8), 110.74 (C-4), 118.93 (C-3'), 122.54 (C-1), 123.82 (C-6), 123.97 (C-1'), 129.03 (C-5), 130.46 (C-4'), 131.69 (C-6'), 137.94 (C-5'), 139.26 (C-2), 146.31 (C-3), 151.36 (C-2').
HRMS für C₁₆H₁₈O₃ (ESI+) [M+Na⁺]: ber: 281.1154, gef.: 281.1157
MS (EI, GCMS): m/z(%): 258 (100) [M]⁺˙, 243 (10) [M-CH₃˙]⁺.
Elementaranalyse für C₁₆H₁₈O_{3:} ber: 74.39%, H: 7.02%, gef.: C: 74.32%, H: 7.20%

### 2,2'-Dihydroxy-5'-isopropyl-3-methoxy-5-methylbiphenyl

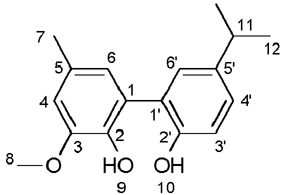

Es wurden 0.69 g (5 mmol, 1.0 Äquiv.) 4-Methylguajacol und 2.05 g (15 mmol, 3.0 Äquiv.) 4-Isopropylphenol in 27 mL HFIP und 6 mL MeOH gelöst, 0.68 g Methyltriethylammoniummethylsulfat (MTES) zugegeben und der Elektrolyt in die Elektrolysezelle überführt. Das Lösungsmittel sowie nicht umgesetzte Eduktmengen werden nach der Elektrolyse unter vermindertem Druck entfernt, das Rohprodukt an Kieselgel 60 als "Flashchromatographie" im Laufmittel 4:1 (Cyclohexan:Essigsäureethylester) aufgereinigt und das Produkt als bräunliches Öl erhalten.
Ausbeute: 0.53 g (39%, 1.9 mmol)

GC (Methode *hart,* HP-5): t_{R}= 14.23 min
R_{f}(CH:EE= 4:1)= 0.30
¹H-NMR (400 MHz, CDCl₃) δ= 1.27 (m, 6H), 2.36 (s, 3H), 2.91 (dt, J= 13.8, 6.9, 6.9 Hz, 1H), 3.94 (s, 3H), 6.13-6.27 (m, 2H), 6.82-6.65 (m, 1 H), 6.25 (m, 2H), 6.75 (s, 1 H), 6.77 (s, 1 H), 6.99 (d, J= 8.1 Hz, 1H), 7.19-7.12 (m, 2H);
¹³C-NMR (101 MHz, CDCl₃) δ= 21.25, 24.27, 33.40, 56.18, 110.92, 117.60, 123.91, 124.23, 125.07, 127.29, 128.80, 130.57, 139.29, 141.42, 146.31, 151.51.
HRMS für C₁₇H₂₀O₃ (ESI+) [M+Na⁺]: ber: 295.1310, gef.: 295.1297
MS (EI, GCMS): m/z(%): 272 (80) [M]⁺˙, 257 (100) [M-CH₃˙]⁺.

### 2,2'-Dihydroxy-3-methoxy-5-methyl-5'-tert-butylbiphenyl

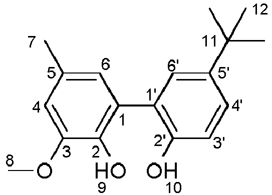

Es wurden 0.69 g (5 mmol, 1.0 Äquiv.) 4-Methylguajacol und 2.26 g (15 mmol, 3.0 Äquiv.) 4-*Tert*-butylphenol in 27 mL HFIP und 6 mL MeOH gelöst, 0.68 g Methyltriethylammoniummethylsulfat (MTES) zugegeben und der Elektrolyt in die Elektrolysezelle überführt. Das Lösungsmittel sowie nicht umgesetzte Eduktmengen werden nach der Elektrolyse unter vermindertem Druck entfernt, das Rohprodukt an Kieselgel 60 als "Flashchromatographie" im Laufmittel 4:1 (Cyclohexan:Essigsäureethylester) aufgereinigt und das Produkt als gelbliches Öl erhalten.
Ausbeute: 0.48 g (34%, 1.7 mmol)

GC (Methode *hart,* HP-5): t_{R}= 14.52 min
R_{f}(CH:EE= 4:1)= 0.24
¹H-NMR (400 MHz, CDCl₃) δ= 1.34 (s, 9H), 2.37 (s, 3H), 3.94 (s, 3H), 6.17 (s, 1H), 6.24 (s, 1H), 6.75 (s, 1 H), 6.77 (s, 1 H), 6.99 (d, J= 8.4 Hz, 1 H), 7.31-7.29 (m, 1 H), 7.33 (dd, J= 8.4, 2.5 Hz, 1 H).
¹³C-NMR (101 MHz, CDCl₃) δ= 21.28, 31.61, 34.20, 56.18, 110.91, 117.25, 123.92, 124.41, 124.63, 126.38, 127.78, 130.58, 139.32, 143.70, 146.32, 151.22.
HRMS für C₁₈H₂₂O₃ (ESI+) [M+Na⁺]: ber: 309.1467, gef.: 309.1476
MS (EI, GCMS): m/z(%): 286 (28) [M]⁺˙, 271 (100) [M-CH₃˙]⁺.

### 2,2'-Dihydroxy-3',5'-di-tert-butyl-5-methyl-3-methoxybiphenyl

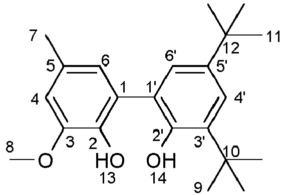

Es wurden 0.69 g (5 mmol, 1.0 Äquiv.) 4-Methylguajacol und 3.12 g (15 mmol, 3.0 Äquiv.) 2,4-Di-*tert*-butylphenol in 27 mL HFIP und 6 mL MeOH gelöst, 0.68 g Methyltriethylammoniummethylsulfat (MTES) zugegeben und der Elektrolyt in die Elektrolysezelle überführt. Das Lösungsmittel sowie nicht umgesetzte Eduktmengen werden nach der Elektrolyse unter vermindertem Druck entfernt, das Rohprodukt an Kieselgel 60 als "Flashchromatographie" im Laufmittel 9:1 (Cyclohexan:Essigsäureethylester) aufgereinigt und das Produkt als farbloser Feststoff erhalten.
Ausbeute: 0.41 g (24%, 1.2 mmol)

GC (Methode*hart*, HP-5): t_{R}= 15.15 min
R_{f}(CH:EE= 9:1)= 0.35
mₚ= 120.2 °C (inn-Pentan umkristallisiert)
¹H-NMR (400 MHz, CDCl₃) δ= 1.36 (s, 9H), 1.50 (s, 9H), 2.38 (s, 3H), 3.96 (s, 3H), 6.00 (s, 1H), 6.05 (s, 1 H), 6.77 (s, 1 H), 7.16 (d, J= 2.5 Hz, 1 H), 7.39 (d, J= 2.5 Hz, 1 H).
¹³C-NMR (101 MHz, CDCl₃) δ= 21.23, 29.88, 31.69, 34.40, 35.23, 56.17, 111.03, 123.96, 124.17, 125.09, 125.50, 130.42, 136.73, 139.72, 142.36, 146.45, 149.82.
MS (EI, GCMS): m/z(%): 342 (22) [M]⁺˙, 327 (100) [M-CH₃˙]⁺.

### 2,2'-Dihydroxy-3',5-dimethyl-3-methoxy-5'-tert-butylbiphenyl

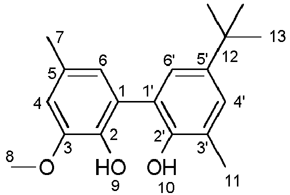

Es wurden 0.69 g (5 mmol, 1.0 Äquiv.) 4-Methylguajacol und 2.47 g (15 mol, 3.0 Äquiv.) 2-Methyl-4-*tert*-butylphenol in 27 mL HFIP und 6 mL MeOH gelöst, 0.68 g Methyltriethylammoniummethylsulfat (MTES) zugegeben und der Elektrolyt in die Elektrolysezelle überführt. Das Lösungsmittel sowie nicht umgesetzte Eduktmengen werden nach der Elektrolyse unter vermindertem Druck entfernt, das Rohprodukt an Kieselgel 60 als "Flashchromatographie" im Laufmittel 4:1 (Cyclohexan:Essigsäureethylester) aufgereinigt und das Produkt als gelbliches Öl erhalten.
Ausbeute: 0.69 g (46%, 2.3 mmol)

GC (Methode *hart,* HP-5): t_{R}= 14.79 min
R_{f}(CH:EE= 4:1)= 0.33
¹H-NMR (400 MHz, CDCl₃) δ= 1.37 (s, 9H), 2.39 (d, J= 2.4 Hz, 6H), 3.94 (s, 3H), 6.15 (s, 1H), 6.17 (s, 1 H), 6.77 (s, 1 H), 6.79 (s, 1 H), 7.17 (d, J= 2.5 Hz, 1 H), 7.24 (d, J= 2.4 Hz, 1 H); ¹³C-NMR (101 MHz, CDCl₃) δ= 16.90, 21.28, 31.67, 34.12, 56.16, 110.94, 124.02, 124.17, 124.59, 125.41, 125.65, 127.86, 130.47, 139.50, 143.07, 146.40, 149.41.
MS (EI, GCMS): m/z(%): 300 (18) [M]⁺˙, 285 (100) [M-CH₃˙]⁺.

### 2,2'-Dihydroxy-3-methoxy-5-methyl-5'-(1-methylethyl)biphenyl

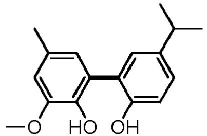

Es wurden 0.69 g (5 mmol, 1.0 eq.) 4-Methylguajacol und 2.05 g (15 mmol, 3.0 eq.) 4-isopropylphenol und 0.68 g Methyltriethylammoniummethylsulfat (MTES) in 27 mL HFIP + 6 mL MeOH Methyltriethylammoniummethylsulfat (MTES) zugegeben und der Elektrolyt in die Elektrolysezelle überführt. Das Lösungsmittel sowie nicht umgesetzte Eduktmengen werden nach der Elektrolyse unter vermindertem Druck entfernt, das Rohprodukt an Kieselgel 60 als "Flashchromatographie" im Laufmittel 4:1 (Cyclohexan:Essigsäureethylester) aufgereinigt und das Produkt als bräunliches Öl erhalten.
Ausbeute: 39%, 527 mg, 1.9 mmol.
R_{f} (Cyclohexane:Ethylacetat = 4:1)= 0.30; ¹H NMR (400 MHz, CDCl₃) δ= 1.27 (m, 6H), 2.36 (s, 3H), 2.91 (sept, J= 6.9 Hz, 1 H), 3.94 (s, 3H), 6.13-6.27 (m, 2H), 6.65-6.82 (m, 2H), 6.99 (d, *J=* 8.1 Hz, 1H), 7.12-7.19 (m, 2H); ¹³C NMR (101 MHz, CDCl₃) δ= 21.37, 24.39, 33.53, 56.31, 111.04, 117.73, 124.04, 124.36, 125.20, 127.42, 128.93, 130.70, 139.42, 141.55, 146.44, 151.64. HRMS für C₁₇H₂₀O₃ (ESI+) [M+Na⁺]: calc.: 295.1310, found: 295.1297; MS (EI, GCMS): m/z(%): 272 (80) [M]⁺˙, 257 (100) [M-CH₃˙]⁺.

### 2,2'-Dihydroxy-3-methoxy-5-methyl-4'-(methylethyl)biphenyl

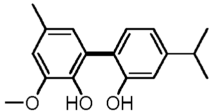

Es wurden 0.69 g (5 mmol, 1.0 eq.) 4-Methylguajacol und 2.065 g (15 mmol, 3.0 eq.) 3-isopropylphenol und 0.68 g Methyltriethylammoniummethylsulfat (MTES) in 33 mL HFIP gelöst und der Elektrolyt in die Elektrolysezelle überführt. Das Lösungsmittel sowie nicht umgesetzte Eduktmengen werden nach der Elektrolyse unter vermindertem Druck entfernt, das Rohprodukt an Kieselgel 60 als "Flashchromatographie" im Laufmittel 4:1 (Cyclohexan:Essigsäureethylester) aufgereinigt und das Produkt als bräunliches Öl erhalten (Ausbeute: 52%, 705 mg, 2.6 mmol).
R_{f} (Cyclohexane:Ethylacetat = 4:1)= 0.29; ¹H NMR (400 MHz, CDCl₃) δ= 1 H NMR (400 MHz, CDCl3) δ 1.27 (s, 3H), 1.29 (s, 3H), 2.34 (s, 3H), 2.91 (sept, *J=* 7.0 Hz, 1 H), 3.94 (s, 3H), 6.15 (s, 1 H), 6.35 (s, 1 H), 6.73 (d, *J=* 1.8 Hz, 1 H), 6.75-6.77 (m, 1 H), 6.90 (dd, *J=* 7.9 Hz, 1.8 Hz, 1 H), 6.94 (d, *J=* 1.7 Hz, 1 H), 7.23 (d, *J=* 7.8 Hz, 1 H). ¹³C NMR (101 MHz, CDCl₃) δ= ¹³C NMR (101 MHz, CDCl₃) δ 21.36, 24.02, 33.92, 56.30, 77.16, 110.91, 115.77, 119.56, 122.81, 124.00, 124.08, 130.65, 130.84, 139.38, 146.43, 150.72, 153.54. HRMS für C₁₇H₂₀O₃ (ESI+) [M+Na⁺]: calc.: 295.1310, found: 295.1305; MS (EI, GCMS): m/z(%): 272 (100) [M]⁺˙, 257 (50) [M-CH₃˙]⁺. Elementaranalyse für C₁₇H₂₀O_{3:} calc.: 74.97%, H: 7.40%, found: C: 75.05%, H: 7.36%.

### 2,2'-Dihydroxy-4',5-dimethyl-3-methoxybiphenyl

Es wurden 0.28 g (2 mmol, 1.0 eq.) 4-Methylguaiacol, 1.22 g (6 mmol, 3.0 eq.) 3-Methylphenyl und 0.77 g MTBS 25 mL HFIP gelöst und der Elektrolyt in die Beaker-typ Elektrolysezelle überführt. Das Lösungsmittel sowie nicht umgesetzte Eduktmengen werden nach der Elektrolyse unter vermindertem Druck entfernt, das Rohprodukt an Kieselgel 60 als "Flashchromatographie" im Laufmittel 4:1 (Cyclohexan:Essigsäureethylester) aufgereinigt und führte zu den beiden Kreuzkupplungsprodukten als farbloses und viskoses Öl.

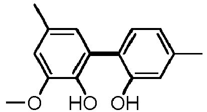

Ausbeute: 21%, 266 mg, 1.1 mmol; R_{f} (Cyclohexane:Ethylacetat = 4:1)= 0.25; mₚ= 136.2 °C (kristallisiert aus Dichloromethan/Cyclohexan); ¹H NMR (400 MHz, CDCl₃) δ= 2.35 (s, 3H), 2.37 (s, 3H,), 3.94 (s, 3H), 6.17 (s, 1 H), 6.35 (s, 1 H), 6.74 (d, *J=* 1.8 Hz, 1 H), 6.76 (s, 1 H), 6.88-6.83 (m, 1H), 6.90 (d, 1 H, *J=* 1.5 Hz), 7.21 (d, 1 H, *J= 7.7* Hz); ¹³C NMR (101 MHz, CDCl₃) δ=21.11, 21.20 56.13, 110.81, 118.25, 121.97, 122.39, 123.77, 123.85, 130.50, 130.68, 139.30, 139.54, 146.31, 153.33. HRMS für C₁₅H₁₆O₃ (ESI+) [M+Na⁺]: calc.: 267.0997, found: 267.1006; MS (EI, GCMS): m/z(%): 244 (100) [M]⁺˙, 229 (18) [M-CH₃˙]⁺,. Elementaranalyse für C₁₅H₁₆O_{3:} calc.: C: 73.75%, H: 6.60%, found: C: 73.70%, H: 6.68%.

### 2,2'-Dihydroxy-5,5'-dimethyl-3'-(1,1-dimethylethyl)-3-methoxybiphenyl

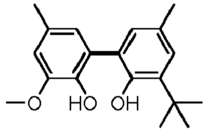

Es wurden 0.69 g (5 mmol, 1.0 eq.) 4-Methylguaiacol, 2.47 g (15 mmol, 3.0 eq.) 4-Methyl-2-*tert*-butylphenol und 0.68 g Methyltriethylammoniummethylsulfat (MTES) in 27 mL HFIP + 6 mL MeOH gelöst gelöst und der Elektrolyt in die Elektrolysezelle überführt. Das Lösungsmittel sowie nicht umgesetzte Eduktmengen werden nach der Elektrolyse unter vermindertem Druck entfernt, das Rohprodukt an Kieselgel 60 als "Flashchromatographie" im Laufmittel 4:1 (Cyclohexan:Essigsäureethylester) aufgereinigt und das Produkt als gelbes Öl erhalten (Ausbeute: 36%, 545 mg, 1.8 mmol).
R_{f} (Cyclohexane:Ethylacetat = 9:1)= 0.36; ¹H NMR (400 MHz, CDCl₃) δ= 1.46 (s, 9H), 2.34 (m, 6H), 3.93 (s, 3H), 5.99 (s, 1H), 6.01 (s, 1H), 6.74 (s, 2H), 6.96 (d, *J=* 1.9 Hz, 1H), 7.14 (d, *J=* 1.9 Hz, 1H); ¹³C NMR (101 MHz, CDCl₃) δ= 21.05, 21.32, 29.96, 35.05, 56.30, 77.16, 111.21, 124.18, 124.24, 125.92, 127.67, 129.15, 129.22, 130.51, 137.57, 139.87, 146.57, 150.10. HRMS für C₂₂H₃₀O₃ (ESI+) [M+Na⁺]: calc.: 323.1623, found: 323.1618; MS (EI, GCMS): m/z(%): 300 (100) [M]⁺˙, 285 (100) [M-CH₃˙]⁺.

Bei den Strukturen (**I**) und **(II)** besitzt je ein Flügel eine Naphthyl-Phenyl-Einheit, bei Struktur **(III)** weisen beide Flügel eine Naphthyl-Phenyl-Einheit auf.

### 1-(2-Hydroxy-3-methoxy-5-methylphenyl)-2-naphthol

Die Durchführung der Elektrolyse erfolgt gemäß der allgemeinen Arbeitsvorschrift in einer ungeteilten Flanschzelle mit BDD- Anode. Hierzu werden 0.78 g (5 mmol, 1.0 Äquiv.) 2-Naphthol und 2.18 g (15 mmol, 3.0 Äquiv.) 4-Methylguajacol in 27 mL HFIP und 6 mL MeOH gelöst, 0.68 g Methyltriethylammoniummethylsulfat (MTES) zugegeben und der Elektrolyt in die Elektrolysezelle überführt. Das Lösungsmittel sowie nicht umgesetzte Eduktmengen werden nach der Elektrolyse unter vermindertem Druck entfernt, das Rohprodukt an Kieselgel 60 als "Flashchromatographie" im Laufmittel 4:1 (CH:EE) aufgereinigt und ein Produktgemisch erhalten. Eine zweite "'Flashchromatographie" in Dichlormethan ermöglicht eine Trennung beider Komponenten als leichtrotes kristallines Haupt- und farbloses kristallines Nebenprodukt.

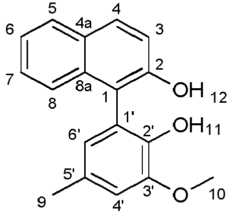

Ausbeute: 899 mg (61%, 3.2 mmol)
GC (Methode *hart,* HP-5): t_{R}= 15.77 min
R_{f}(CH:EE= 4:1)= 0.36, R_{f}(DCM)= 0.36
mₚ= 145.5 °C (aus DCM/CH umkristallisiert)
¹H-NMR (400 MHz, CDCl₃) δ= 2.39 (s, 3H, 9-H), 3.96 (s, 3H, 10-H), 5.47-5.52 (m, 1H, 12-H), 5.65- 5.69 (m, 1 H, 11-H), 6.75 (d, 1 H, 6'-H), 6.85 (d, 1 H, 4'-H), 7.32 (dd, 1 H, 3-H), 7.34-7.43 (m, 2H, 6-H/7-H), 7.51 (d, 1 H, 8-H), 7.83 (s, 1 H, 5-H), 7.85 (d, 1 H, 4-H);
Kopplungen: ³J_{3-H, 4-H}= 9.0 Hz, ³J_{7-H, 8-H}= 8.3 Hz, ⁴J_{4'-H, 6'-H}= 1.8 Hz;
¹³C-NMR (101 MHz, CDCl₃) δ= 21.22 (C-9), 56.08 (C-10), 112.06 (C-4'), 116.62 (C-1), 117.81 (C-3), 119.33 (C-1'), 123.36 (C-6/C-7), 124.42 (C-6'), 124.86 (C-8), 126.48 (C-6/C-7), 128.15 (C-4), 129.18 (C-4a), 129.83 (C-5), 130.36 (C-5'), 133.16 (C-8a), 141.72 (C-2'), 147.24 (C-3'), 150.84 (C-2).
HRMS für C₁₈H₁₆O₃ (ESI+) [M+Na⁺]: ber: 303.0997, gef.: 303.1003
MS (EI, GCMS): m/z(%): 280 (100) [M]⁺˙, 265 (12) [M-CH₃˙]^{+,},249 (12) [M-OCH₃˙]⁺. Elementaranalyse für C₁₈H₁₆O_{3:} ber: C: 77.12%, H: 5.75%, gef.: C: 76.96%, H: 5.82%.

### 1-(3-(Dimethylethyl)-2-hydroxy-5-methoxyphenyl)-2-naphthol

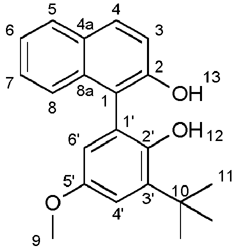

Die Durchführung der Elektrolyse erfolgt gemäß AAV1 in einer ungeteilten Flanschzelle mit BDD-Anode. Hierzu werden 0.72 g (5 mmol, 1.0 Äquiv.) 2-Naphthol und 2.77 g (15 mmol, 3.0 Äquiv.) 2-(Dimethylethyl)-4-methoxyphenol in 27 mL HFIP und 6 mL MeOH gelöst, 0.68 g Methyltriethylammoniummethylsulfat (MTES) zugegeben und der Elektrolyt in die Elektrolysezelle überführt. Das Lösungsmittel sowie nicht umgesetzte Eduktmengen werden nach der Elektrolyse unter vermindertem Druck entfernt, das Rohprodukt an Kieselgel 60 als "Flashchromatographie" im Laufmittel 9:1 (CH:EE) aufgereinigt und das Produkt als farbloser Feststoff erhalten.
Ausbeute: 1.05 g (63%, 3.2 mmol)
GC (Methode *hart,* HP-5): t_{R}= 15.75 min
R_{f}(CH:EE= 4:1)= 0.43
mₚ= 139.9 °C (aus DCM/CH umkristallisiert)
¹H-NMR (400 MHz, CDCl₃) δ= 1.46 (s, 9H, 11-H), 3.77 (s, 3H, 9-H), 4.72 (s, 1 H, 2'-H), 5.36 (s, 1 H, 2-H), 6.63 (d, 1 H, 6'-H), 7.08 (d, 1 H, 4'-H), 7.32 (d 1 H, 3-H), 7.50-7.35 (m, 3H, 6-H/7-H/8-H), 7.87-7.83 (m, 1 H, 5-H), 7.89 (d, 1 H, 4-H);
Kopplungen: ³J_{3-H, 4-H}= 8.9 Hz; ⁴J_{4'-H, 6'-H}= 3.1 Hz;
¹³C-NMR (101 MHz, CDCl₃) δ= 29.41 (C-11), 35.19 (C-10), 55.68 (C-9), 111.95 (C-6'), 114.18 (C-1), 115.87 (C-4'), 117.63 (C-3), 119.16 (C-1'), 123.89, 124.15 (C-6/C-8), 127.38 (C-7), 128.31 (C-5), 129.19 (C-4a), 130.97 (C-4), 132.99 (C-8a), 139.05 (C-3'), 146.93 (C-2'), 151.94 (C-2), 153.41 (C-5').
HRMS für C₂₁H₂₂O₃ (ESI+) [M+Na⁺]: ber: 345.1467, gef.: 345.1465
MS (EI, GCMS): m/z(%): 322 (100) [M]⁺˙, 307 (38) [M-CH₃˙]⁺.
Elementaranalyse für C₂₁H₂₂O_{3:} ber: 78.23%, H: 6.88%, gef.: C: 78.18%, H: 6.82%.

Der Zentral-Baustein weist eine 2,3'-Biphenol-Einheit auf.

### 2,5'-Dihydroxy-4',5-dimethoxy-2'-methylbiphenyl

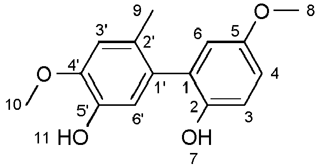

Es wurden 1.66 g (12 mmol, 1.0 Äquiv.) 4-Methylguajacol und 4.49 g (36 mmol, 3.0 Äquiv.) 4-Methoxyphenol in 80 mL HFIP gelöst, 1.63 g MTES zugegeben und der Elektrolyt in die Elektrolysezelle überführt. Das Lösungsmittel sowie nicht umgesetzte Eduktmengen werden nach der Elektrolyse unter vermindertem Druck entfernt, das Rohprodukt an Kieselgel 60 als "Flashchromatographie" im Laufmittel 4:1 (Cyclohexan:Essigsäureethylester) aufgereinigt und das Produkt als farbloser Feststoff erhalten.
Ausbeute: 2.05 g (66%, 7.9 mmol)

GC (Methode *hart,* HP-5): t_{R}= 14.03 min
R_{f}(CH:EE= 4:1)= 0.33
mₚ= 118.7 °C (aus DCM/CH umkristallisiert)
¹H-NMR (600 MHz, DMSO) δ= 2.01 (s, 3H, 9-H), 3.66 (s, 3H, 8-H), 3.77 (s, 3H, 10-H), 6.53 (d, 1 H, 6-H), 6.55 (s, 1 H, 6'-H), 6.72 (dd, 1 H, 4-H), 6.77 (s, 1 H, 3'-H), 6.79 (d, 1 H, 3-H), 8.73 (s, 1 H, 11-H), 8.75 (s, 1H, 7-H);
Kopplungen: ³J_{3-H, 4-H}= 8.7 Hz; ⁴J_{4-H, 6-H}= 3.0 Hz
¹³C-NMR (151 MHz, DMSO) δ= 19.33 (C-9), 55.32 (C-8), 55.73 (C-10), 113.24 (C-4), 113.75 (C-3'), 115.99 (C-3), 116.07 (C-6), 117.40 (C-6'), 126.56 (C-2'), 129.06 (C-1), 130.95 (C-1'), 143.80 (C-5'), 146.52 (C-4'), 148.29 (C-2), 151.81 (C-5).
HRMS für C₁₅H₁₆O₄ (ESI+) [M+Na⁺]: ber: 283.0946, gef.: 283.0942
MS (EI, GCMS): m/z(%): 260 (100) [M]⁺˙, 245 (12) [M-CH₃˙]⁺.
Elementaranalyse für C₁₅H₁₆O_{4:} ber: 69.22%, H: 6.20%, gef.: C: 69.02%, H: 6.34%.

### 3,4'-di-tert-butyl-5,6'-dimethoxy-[1,1'-biphenyl]-2,3'-diol

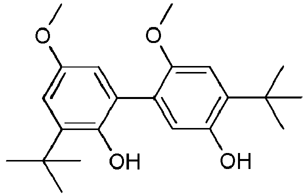

Die Synthese erfolgte nach der obigen allgemeinen Arbeitsvorschrift. Das Produkt konnte nach säulechromatografischer Aufreinung in 15%-iger Ausbeute erhalten werden.
Für einen alternativen Herstellweg siehe: Anwar A. Hamama, Wassef W. Nawar Journal of Agricultural and Food Chemistry 1991, 36, 1063-1069.

### Synthese der Chlorophosphite

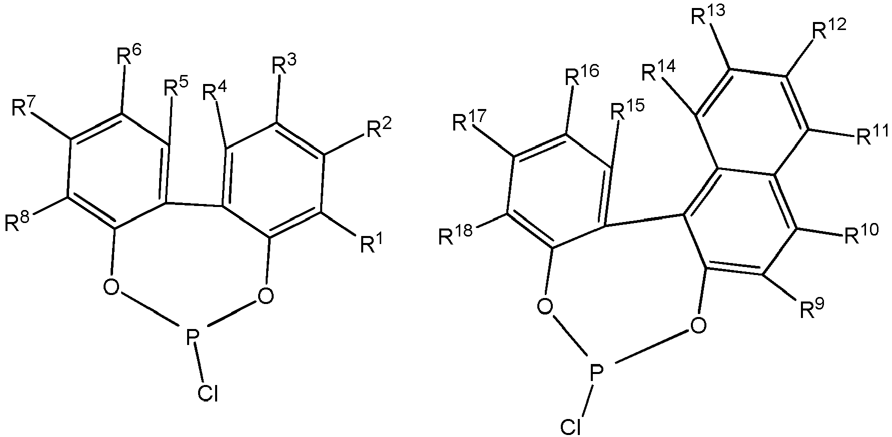

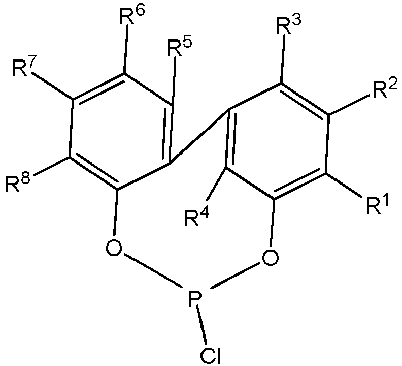

Für die Herstellung der erfindungsgemäßen Verbindungen werden verschieden Chlorophosphite benötigt und zwar einerseits solche, die eine Biphenol-Einheit aufweisen und andererseits solche, die eine Naphthyl-Phenyl-Einheit aufweisen.

Als ein exemplarisches Beispiel sei hier das 6-Chlorodibenzo[*d,f*][1,3,2]dioxaphosphepin genannt, dass sich nach DE 10 2008 043 584 herstellen lässt. Alle anderen Chlorophosphite lassen sich in analoger Weise, d.h. durch Zusatz von Phosphortrichlorit in Gegenwart einer Base, herstellen. Siehe hierzu auch "Phosphorous(III) Ligands in Homogeneous Catalysis - Design and Synthesis" von Paul C.J. Kamer und Piet W.N.M. van Leeuwen; John Wiley and Sons, 2012; unter anderem S. 94 ff. und darin zitierte Literaturstellen.
Da auch Chlorophosphite aus Binol (1,1'-Binaphthol) auf analoge Weise wie solche mit einer Biphenol-Einheit hergestellt werden, werden auch solche Chlorophosphite, die eine Naphthyl-Phenyl-Einheit aufweisen, auf analogem Wege synthetisiert.

### Synthese der erfindungsgemäßen Verbindungen

Die erfindungsgemäßen Verbindungen lassen sich hierbei auf verschiedenen Wegen herstellen. Drei mögliche Wege sind in den nachfolgenden Schemata dargestellt.
Die dargestellten Reaktionswege sind nur exemplarisch und in einer vereinfachten Form dargestellt. So kann nach Bedarf in allen Schritten zusätzlich Base oder Lösungsmittel verwendet werden. Diese sind dem Fachmann hinreichend bekannt und kann der Fachliteratur entnommen werden, wie "Phosphorous(III) Ligands in Homogeneous Catalysis - Design and Synthesis" von Paul C.J. Kamer und Piet W.N.M. van Leeuwen; John Wiley and Sons, 2012; unter anderem S. 94 ff. und darin zitierte Literaturstellen.
Die nachfolgend aufgezeigten Reaktionsschemata stellen Vorschläge da. Die Reihenfolge der Anbindung der einzelnen Bausteine an den Zentralbaustein kann variiert werden, beispielsweise indem zunächst die Naphthyl-Phenyl-Einheit in Form ihres Chlorophosphits angebunden wird, dann die so generierte Struktur mit Phosphortrichlorit umgesetzt wird und anschließend ein Biphenol angebunden wird. Ein solches Vorgehen ist dem Fachmann hinreichend bekannt und kann der Fachliteratur entnommen werden.

Reaktionsschema zu einer Verbindung nach Formel (**I**)

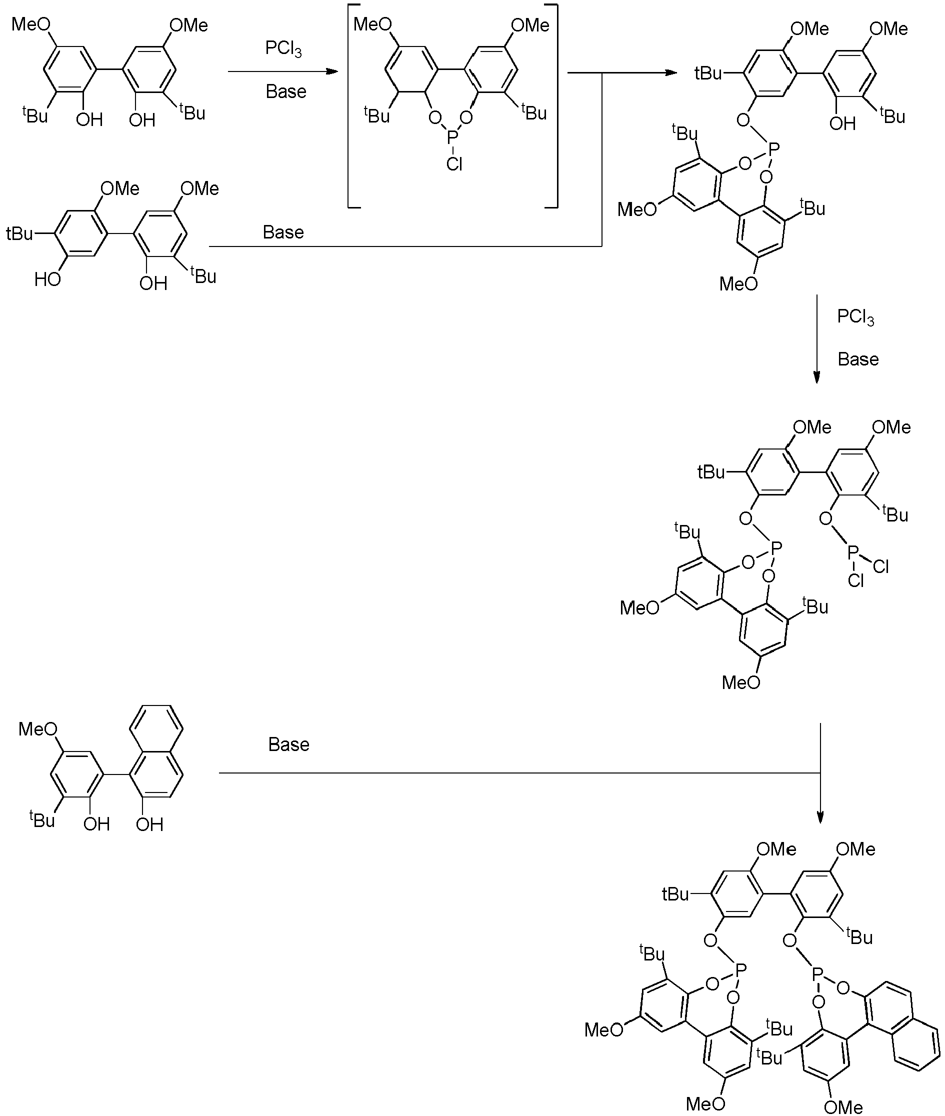

Reaktionsschema zu einer Verbindung nach Formel **(II)**

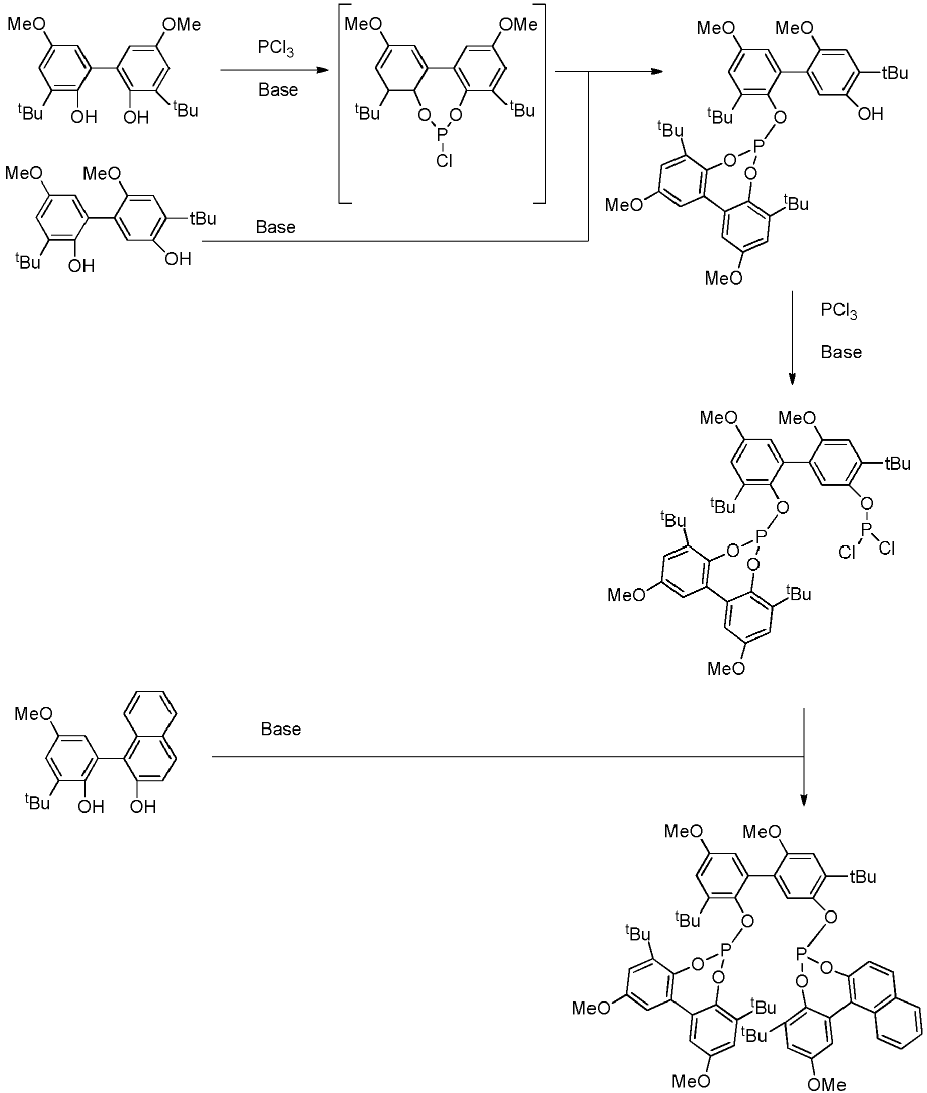

Reaktionsschema zu einer Verbindung nach Formel **(III)**

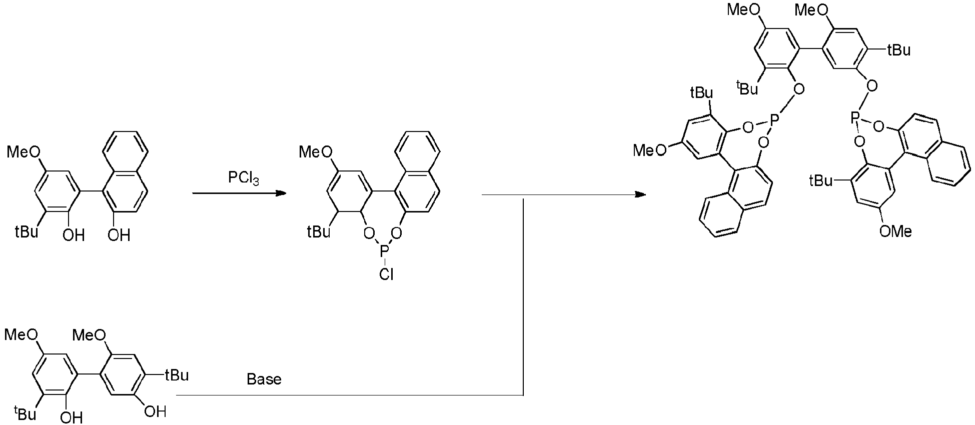

Es konnte erstmalige eine Syntheseroute aufgezeigt werden, mit welcher die erfindungsgemäßen Verbindunen auf einem effizienten Weg hergestellt werden können. Die hierdurch erhaltenen Bisphosphite erfüllen die Aufgabenstellung, da diese erstmalig eine Naphthyl-Phenyl-Einheit als Flügel-Baustein und eine 2,3'-Biphenol-Einheit als Zentral-Baustein aufweisen.

## Patentansprüche

1. Verbindung, welche eine der drei allgemeinen Strukturen (**I**) bis **(III)** aufweist: 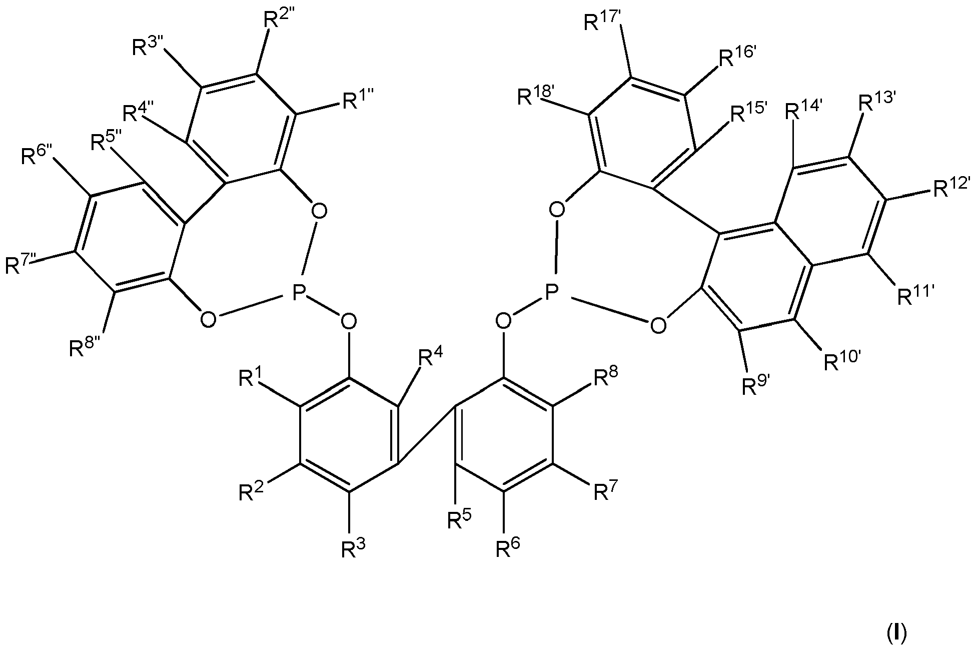 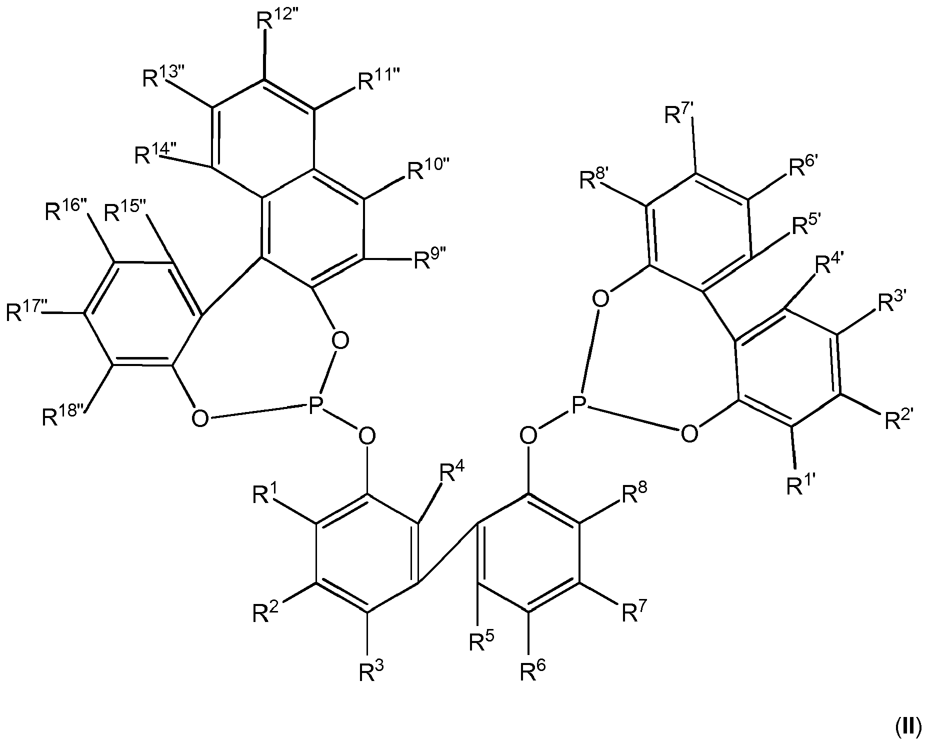 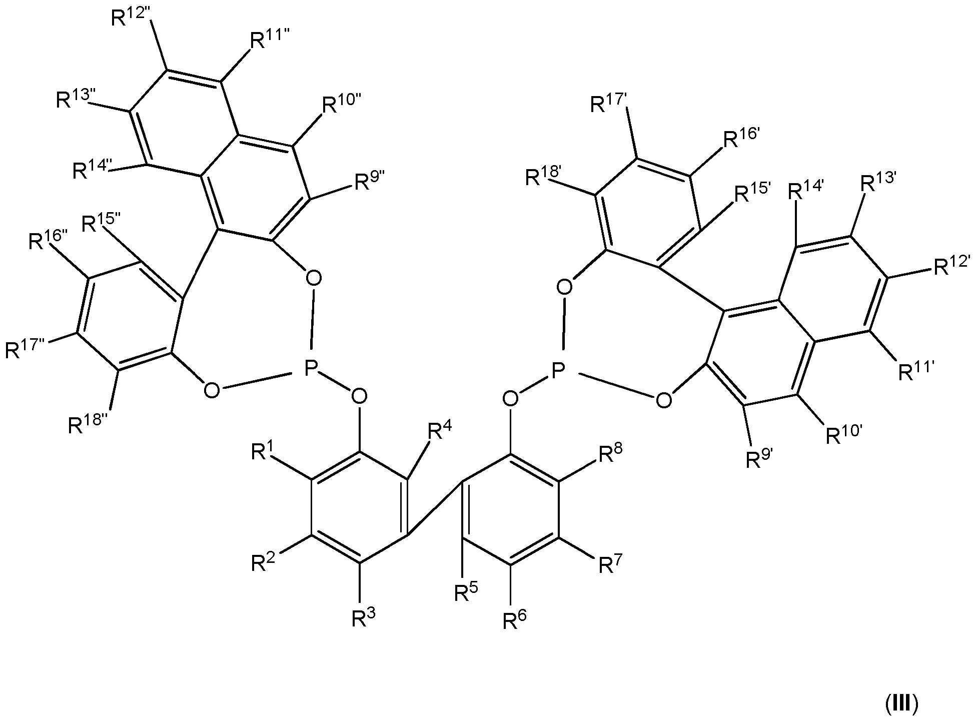 wobei
R¹, R², R³, R⁴, R⁵, R⁶, R⁷, R⁸ ausgewählt sind aus:
- H, -(C₁-C₁₂)-Alkyl, -O-(C₁-C₁₂)-Alkyl, -O-(C₆-C₂₀)-Aryl, -(C₆-C₂₀)-Aryl, -S-Alkyl, -S-Aryl, Halogen, COO-(C₁-C₁₂)-Alkyl, CONH-(C₁-C₁₂)-Alkyl, , -CO-(C₁-C₁₂)-Alkyl, -CO-(C₆-C₂₀)-Aryl, -COOH, -OH, -SO₃H, -CN, -NH₂, -N[(C₁-C₁₂)-Alkyl]₂;
R¹', R²', R³', R⁴', R⁵', R⁶', R⁷', R⁸', R⁹', R¹⁰', R¹¹', R¹²', R¹³', R¹⁴', R¹⁵', R¹⁶', R¹⁷', R¹⁸' ausgewählt sind aus:
-H, -(C₁-C₁₂)-Alkyl, -O-(C₁-C₁₂)-Alkyl, -O-(C₆-C₂₀)-Aryl, -(C₆-C₂₀)-Aryl, -S-Alkyl, -S-Aryl, Halogen, COO-(C₁-C₁₂)-Alkyl, CONH-(C₁-C₁₂)-Alkyl, -CO-(C₁-C₁₂)-Alkyl, -CO-(C₆-C₂₀)-Aryl, -COOH, -OH, - SO₃H, -NH₂, -N[(C₁-C₁₂)-Alkyl]₂;
R¹", R²", R³", R⁴", R⁵", R⁶", R⁷", R⁸", R⁹", R¹⁰", R¹¹", R¹²", R¹³", R¹⁴", R¹⁵", R¹⁶", R¹⁷", R¹⁸" ausgewählt sind aus:
-H, -(C₁-C₁₂)-Alkyl, -O-(C₁-C₁₂)-Alkyl, -O-(C₆-C₂₀)-Aryl, -(C₆-C₂₀)-Aryl, -S-Alkyl, -S-Aryl, Halogen, COO-(C₁-C₁₂)-Alkyl, CONH-(C₁-C₁₂)-Alkyl, -CO-(C₁-C₁₂)-Alkyl, -CO-(C₆-C₂₀)-Aryl, -OH, -SO₃H,-NH₂, -N[(C₁-C₁₂)-Alkyl]₂;
wobei die genannten Alkyl- und Arylgruppen substituiert sein können.

2. Verbindung nach Anspruch 1,
wobei R¹, R², R³, R⁴, R⁵, R⁶, R¹, R⁸ ausgewählt sind aus:
-H, -(C₁-C₁₂)-Alkyl, -O-(C₁-C₁₂)-Alkyl, -O-(C₆-C₂₀)-Aryl, -S-Alkyl, -S-Aryl.

3. Verbindung nach einem der Ansprüche 1 oder 2,
wobei R¹', R²', R³', R⁴', R⁵', R⁶', R⁷', R⁸' ausgewählt sind aus:
-H, -(C₁-C₁₂)-Alkyl, -O-(C₁-C₁₂)-Alkyl, -O-(C₆-C₂₀)-Aryl, -S-Alkyl, -S-Aryl.

4. Verbindung nach einem der Ansprüche 1 bis 3,
wobei R¹", R²", R³", R⁴", R⁵", R⁶", R⁷", R⁸" ausgewählt sind aus:
-H, -(C₁-C₁₂)-Alkyl, -O-(C₁-C₁₂)-Alkyl, -O-(C₆-C₂₀)-Aryl, -S-Alkyl, -S-Aryl.

5. Verbindung nach einem der Ansprüche 1 bis 4,
wobei R⁹', R¹⁰' R¹¹', R¹²', R¹³', R¹⁴', R¹⁵', R¹⁶', R¹⁷', R¹⁸' ausgewählt sind aus:
-H, -(C₁-C₁₂)-Alkyl, -O-(C₁-C₁₂)-Alkyl, -O-(C₆-C₂₀)-Aryl, -S-Alkyl, -S-Aryl.

6. Verbindung nach einem der Ansprüche 1 bis 5,
wobei R⁹", R¹⁰", R¹¹", R¹²" R¹³", R¹⁴", R¹⁵", R¹⁶", R¹⁷", R¹⁸" ausgewählt sind aus:
-H, -(C₁-C₁₂)-Alkyl, -O-(C₁-C₁₂)-Alkyl, -O-(C₆-C₂₀)-Aryl, -S-Alkyl, -S-Aryl.

7. Verbindung nach einem der Ansprüche 1 bis 6,
wobei die Verbindung die allgemeine Struktur (I) aufweist.

8. Verbindung nach einem der Ansprüche 1 bis 6,
wobei die Verbindung die allgemeine Struktur **(II)** aufweist.

9. Verbindung nach einem der Ansprüche 1 bis 6,
wobei die Verbindung die allgemeine Struktur **(III)** aufweist.
